# EUROPEAN PATENT APPLICATION

(11) **EP 4 122 425 A1**
(43) Date of publication of application: **25.01.2023**
(21) Application number: 21187506.7
(22) Date of filing: 23.07.2021
(51) Int. Cl.: A61F 2/24

(54) **A FIXATION DEVICE FOR IMPLANTATION**

(71) Applicant: The Provost, Fellows, Foundation Scholars, and the other members of Board, of the College of the Holy & Undiv. Trinity of Queen Elizabeth near Dublin, Dublin 2 (IE)
(72) Inventor: O'Connor, Eimear, Co. Dublin, Dublin 2 (IE); Murphy, Bruce, Co. Dublin, Dublin 2 (IE)
(74) Representative: Tomkins & Co

(57) **Abstract**

A fixation device for fastening to a native heart valve having native valve leaflets, the fixation device comprising:
(a) a device body, and
(b) at least one clamp on the device body, the clamp for engaging and holding a native valve leaflet, the clamp comprising at least one moveable jaw, the moveable jaw having thereon at least one gripping element which is moveable relative to the moveable jaw between a native valve leaflet gripping configuration of the moveable jaw where the gripping element is arranged to grip a native valve leaflet and a passive configuration of the moveable jaw where the gripping element is arranged so that it cannot grip a native valve leaflet. This provides a device that can be repositioned during a medical procedure.

## Description

### Field of the Invention

The present invention relates to a fixation device for implantation. For example it may be a fixation device for fastening to tissue such as a native heart valve having native valve leaflets. It may be configured as a stent or a valve. It may be configured as a stent with a valve mounted thereon/therein. Of particular interest is a device of the invention in the form of a prosthetic valve.

### Background to the Invention

The use of fixation devices within the body, for example within the vasculature, is well known. For example it is well known to provide replacement artificial valves in the heart when native valve(s), for example mitral or tricuspid valve, are dysfunctional. Artificial valves can repair or replace lost valve function.

Examples of such devices are described for example in patent publications US2011/137397 (Edwards Lifesciences); US2016/324633 (Mitraltech), EP3654886 (Murphy et al), US Patent No. 7,635,329 to Evalve Inc., and US Patent No. 10,905,552 (Edwards Lifesciences).

Notwithstanding devices provided in the past, it remains a challenge to provide a fixation device that can be implanted and secured in an optimal position. For example, during a procedure, optimal positioning may be difficult to achieve especially on a first attempt and re-positioning of the device one or more times may be required during an implantation procedure. So the device has to allow for re-positioning yet still adequately fix itself in place when a final position is selected. This is especially the case for devices implanted percutaneously.

In the case of a fixation device used to fix an artificial valve in place, the issue that arises is, that correct positioning of the device, to allow the artificial valve to repair or replace function of a native valve, is necessary. Accordingly for artificial valves the positioning of the fixation device and in particular a valve it carries is critical.

An additional issue that arises, either during the procedure or after the procedure, is that the fixation strength of the implant's gripping arrangement can be limited and detachment of the implant from the leaflets can occur either during a procedure or after a procedure. The gripping arrangement of the current devices, the Mitraclip and the Pascal device involve short friction elements.

A further issue arising (and irrespective of correct positioning) is that the surgeon carrying out the procedure may not be able to tell if the fixation device has a secure positioning. For example, a surgeon may not be able to tell if a secure grip of native tissue, for example native leaflets, has been achieved. This is a significant issue, as both the position of the device and how well it is held in place are key to a successful procedure and a long-term successful outcome for the patient. If a device is deployed in a correct position but is not securely held in place this means it will move out of position over time. On the other hand if the device is deployed in a permanent position which later turns out to be suboptimal then a further, typically invasive, procedure may be required to remove it, including, for example, open heart surgery.

Notwithstanding all of the solutions proposed to date it remains an objective to provide an implant device that can be secured in place within the body but which allows for ease of repositioning if required. Also it is desirable to provide a device that will be retained securely in place. Another desire is to allow the device to be delivered with a minimal invasiveness. If a device is to be implanted with minimal invasiveness then the device suitably is capable of being delivered percutaneously and typically by transcatheter. It is particularly desirable to provide such a device that can remain in place despite being subject to natural movement of the body. For example a device used to repair or replace heart valve function must be capable of remaining in place despite the motion of a beating heart and associated blood flow.

The present invention addresses these challenges by allowing repositioning, yet locking securely in a finally selected position, and is capable of being delivered percutaneously and typically by transcatheter.

### Summary of the Invention

The present invention relates to implant devices for native valves within the human or animal body. Of particular interest are heart valves. The device of the invention is for use with valves that are dysfunctional and in particular for use with valves which do not close properly or otherwise allow blood flow through the valve when closed, for example due to perforation. In other cases the valve may close but the annulus across which it closes is dysfunctional, for example it may be dilated. Such dysfunctional valves are often referred to as leaky/leaking valves. Valves that do not close properly impair the pumping function of the heart as blood pumped by the heart can leak back through the valve instead of being pumped out through the vasculature. The present invention is particularly useful for dysfunctional mitral valves. While the present invention is described in relation to the mitral valve it will be appreciated that it is intended for application to other valves such as the tricuspid valves.

In one aspect, the present invention provides a fixation device for fastening to a native heart valve having native valve leaflets, the fixation device comprising:
(a) a device body, and
(b) at least one clamp on the device body, the clamp for engaging and holding a native valve leaflet, the clamp comprising at least one moveable jaw, the moveable jaw having thereon at least one gripping element which is moveable relative to the moveable jaw between a native valve leaflet gripping configuration of the moveable jaw where the gripping element is arranged to grip a native valve leaflet and a passive configuration of the moveable jaw where the gripping element is arranged so that it cannot grip a native valve leaflet.

A device of the invention is reversibly moveable from its passive configuration to its gripping configuration and vice versa so that a device of the invention can be positioned and then repositioned if necessary. This allows much greater flexibility to a user to ensure the device is correctly positioned. In particular if there is not sufficient capture of one or more leaflets the user can release one or both leaflets and then recapture.

A device of the invention can have a locking mechanism to lock to prevent movement from the gripping configuration to the passive configuration and vice versa. This means that once a user is satisfied with the implantation they can lock the device in place.

Desirably the device is configured so that movement of the moveable jaw modulates movement of the gripping element relative to the moveable jaw. This means that movement of the jaw can also effect movement of the gripping element so one action controls two functions. For example the device may automatically retract or raise the gripping element(s) with movement of the moveable jaw.

In one simple construction the moveable jaw is pivotably mounted on the device body by a pivot and moves by pivotable movement about the pivot. In such an arrangement the movement about the pivot can be used to effect movement to the gripping element(s) also.

Desirably the device has a clamp closed position and a first clamp open position and the moveable jaw has a first range of movement from the clamp closed position to the first clamp open position through which the jaw is in its native valve gripping configuration. This is a range of movement through which the device is in a capture ready position or gripping position. In this arrangement it can catch and then close to hold a leaflet. This is a range of movement where the gripping elements can capture tissue.

The moveable jaw suitably has a second range of movement (distinct from the first range of movement) from the first clamp open position to a second clamp open position, wherein in the second clamp open position the moveable jaw is in its passive configuration where the gripping element is arranged so that it cannot grip a native valve leaflet. For example in such a range of movement the gripping elements may be moved from their gripping configuration to their passive configuration. This means that as the device opens further it becomes passive and for example will not inadvertently catch on any tissue for example heart or valve tissue. The passive configuration is one in which the device can be safely moved about while trying to correctly position it.

For example the device may be configured so that during movement through the second range of movement the gripping element(s) is automatically progressively moved from where it is arranged to grip a native valve leaflet to where it is arranged so that it cannot grip a native valve leaflet. For example this automatic progressive movement may be correlated to the pivoting movement of the moveable jaw.

Desirably the moveable jaw has a third range of movement (distinct from the first range of movement and the second range of movement) from the second open position to a third open position, through which the moveable jaw is in its passive configuration. In relation to the present invention this may be an inverted position as described below.

A part of the gripping element(s), for example a gripping surface of the gripping element(s), stands proud of the (clamping face of the) moveable jaw in the native valve leaflet gripping configuration of the moveable jaw. This is a capture ready position of the gripping element(s).

Desirably, in the passive configuration of the moveable jaw, no part of the gripping element stands proud of the (clamping face of the) moveable jaw. As above this means that the device will not catch on any tissue during movement to a target implantation site. Also if repositioning is desired the device can be moved back to its passive configuration one or more times to assist with repositioning. And of course where there is one or more clamps on the device of the invention they can be operated independently. This means that if only one moveable arm needs to be repositioned this can be achieved while leaving the other moveable arm unmoved.

Desirably the position of the gripping element relative to the moveable jaw is changed by raising or lowering the gripping element relative to the jaw. For example the position of the gripping element relative to the moveable jaw is changed by pivoting the gripping element relative to the jaw.

The position of the gripping element relative to the moveable jaw may be changeable by depression of the gripping element, optionally by depression against the biasing force of a biasing means.

Suitably the position of the gripping element relative to the moveable jaw is changeable by deforming the gripping element, optionally wherein the gripping element is resiliently deformable for example by being formed of a resiliently deformable material, e.g. a material such as nitinol. Such an arrangement is desirable because deflection of the gripping element(s) can be used to move them between their gripping configuration and their passive configuration. Deflection may be imparted by the pivoting action of the moveable jaw.

The moveable jaw has a gripping face, and optionally when the gripping element is in the native valve leaflet gripping configuration, the gripping element is aligned with the gripping face and when the gripping element is in the passive configuration the gripping element is arranged so that it is displaced relative to the gripping face.

Desirably the device has a mechanism for changing the position of the gripping element relative to the moveable jaw, desirably a mechanism for automatically changing the position of the gripping element relative to the moveable jaw.

Optionally the mechanism comprises a cam surface and movement of the moveable jaw causes the cam surface to impart a force to the gripping element to move it relative to the moveable jaw.

A device of the invention may further comprise a sliding element which slides on and relative to the moveable jaw and which is slideable in a first direction across the gripping element to move the gripping element relative to the moveable jaw; optionally by depressing the gripping element against a resilient bias. The sliding element is desirably moved by the pivoting action of the moveable arm.

Suitably sliding movement of the sliding element in the first direction causes the gripping element to depress, optionally against the resilient bias, and wherein sliding movement of the sliding element in a second (opposite) direction allows the gripping element to move with the resilient bias.

Desirably the gripping element projects through the sliding element. This means that the movement of the sliding element can control the gripping element(s) in two opposing directions. For example deflect/depress it/them in one direction and in the other direction abut the gripping element(s) for example to hold them in a gripping configuration such as by preventing them depressing when capturing tissue.

Desirably sliding movement of the sliding element in the first direction causes the gripping element to be concealed by the sliding element. Optionally sliding movement of the sliding element in a second direction causes the gripping element to be revealed.

Desirably the gripping element is resiliently biased and the resilient bias is sufficient to resiliently bias the sliding element in a second direction, opposite to the first direction. Additionally or alternatively the sliding element itself may be resiliently deformable. For example it may be resiliently compressible and return to its uncompressed state when force is removed for example to depress/deflect the gripping element(s) it may be resiliently compressed but allow the gripping elements to return to their gripping configuration when the resilient compression is removed.

Suitably the device has a cam surface and movement of the moveable jaw causes the cam surface to impart, in the first direction, a sliding movement to the sliding element.

Optionally the sliding movement imparted by the cam surface acts against the resilient bias.

The moveable jaw may be pivotable about a pivot relative to the device body and optionally the movement of the jaw that causes the cam surface to impart, in the first direction, a sliding movement to the sliding element, is a pivoting movement of the moveable jaw about the pivot. Again this is a simple yet effective arrangement.

The clamp is desirably formed by the moveable jaw and a non-moving part of the device body, for example a non-moving jaw. Desirably there are at least two clamps optionally on opposing sides of the device of the invention with the stent element located between them.

Desirably the gripping element projects into the non-moveable part. This provides secure capture of tissue.

Desirably the moveable jaw moves by pivoting about a pivot from the closed position to the first, second and third open positions and the second open position is at least 110 degrees of rotation away (of the moveable jaw) from the closed position. This means the device is adapted to automatically and progressively move from gripping to the passive configurations during opening of the moveable jaw and of course the reverse is true also.

Desirably the second open position is at least 120 degrees of rotation away from the closed position. This allows for capture of tissue.

The gripping element(s) may be curved for example a hook shape. This means the gripping element(s) may face in a desired direction.

The gripping element may be a piercing element. In such an arrangement it pierces the captured tissue. Piercing includes partial penetration into the tissue. Desirably it does not extend the whole way through the captured tissue but instead partially penetrates it. For example in the native valve leaflet gripping configuration of the moveable jaw the piercing element may be arranged to pierce a native valve leaflet, and in the passive configuration of the moveable jaw the piercing element is arranged so that it cannot pierce a native valve leaflet.

Instead of, or in addition to the sliding element, the device may further comprise a cable and movement of the moveable jaw causes the cable to impart a force, for example impart a tension force in the cable, to the gripping element to move it relative to the moveable jaw. This may be achieved by having the cable impart a force on the gripping element(s) for example it may be attached to each gripping element. Of course the cable can additionally or alternatively be operable independent of the movement of the moveable jaw.

Where the gripping element(s) are resiliently deformable, force imparted by the cable may cause the gripping element(s) to be depressed/deflected. When the force is removed then the gripping element(s) will return to their original position. Typically resilient deformation/deflection of the gripping element(s) causes them to move toward the passive configuration.

Additionally or alternatively the gripping element(s) may move between the passive and gripping configurations. For example they could be arranged to pivot between the passive and gripping configurations. For example they could be biased by biasing means such as a spring towards the gripping configuration or towards the passive configuration. Or indeed may not have any biasing means. Biasing may cause them to pivot towards a desired position when force countering the biasing action is not present.

In all embodiments of the invention a pivoting movement of the moveable jaw may cause the cable to impart a force, for example impart a tension force in the cable, to the gripping element(s) to move it relative to the moveable jaw.

The present invention also provides a fixation device for fastening to a native heart valve having native valve leaflets, the fixation device comprising:
(i) a device body,
(ii) at least one clamp on the device body, the clamp for engaging and holding a native valve leaflet, the clamp comprising at least one jaw, the jaw having thereon at least one gripping element, the gripping element being resiliently deformable,
(iii) a contact on the device body; and
the gripping element and the contact being arranged so that when the clamp grips a native valve leaflet, deformation of the gripping element occurs causing the gripping element to move against the contact.

This is a simple construction where the movement against the contact occurs only when sufficient tissue is gripped. This then provides an indicator whether or not a user has successfully captured a valve leaflet.

Desirably the jaw is a moveable jaw. Desirably the gripping element is moveable relative to the jaw between a native valve leaflet gripping configuration of the jaw where the gripping element is arranged to grip a native valve leaflet and a passive configuration of the jaw where the gripping element is arranged so that it cannot grip a native valve leaflet.

This fixation device of the invention is not distinct from other devices of the invention insofar as features of this fixation device and features of other fixation devices described herein can be combined in any combination. In particular this fixation device can have any of the constructions set out herein and in the claims.

The present invention also provides an implant device for implantation into the animal body, the implant comprising:
(i) a device body;
(ii) at least one moveable securing element (for example a moveable jaw as described above) on the device body which is moveable relative to the device body for securing the device in position by gripping tissue;
(iii) at least one sensor on the device body or the securing element for indicating that the securing element has gripped tissue;
wherein the at least one sensor is detachable so that it is removable after the device has been implanted.

This is a very desirably construction because once a device is implanted the sensor can be removed leaving a more simple fixation device within the body. This means there is less features on the implant device to deteriorate over time.

Desirably the at least one sensor has an electrical wire electrically connected thereto and the sensor is detachable by applying a force such as a tensile force to the electrical wire. The sensor may be of any suitable kind including those described herein. In addition to being useful to remove the senor the electrical wire may also power the sensor.

Being removable the sensor(s) and any associated wiring can be removed for example through an introducer/delivery catheter. The sensor(s) can be removed through the same means as those use to introduce the device into the body.

A series of sensors can be provided for indication of capture of tissue along the device.

The at least one sensor may be positioned in a recess in the device body or the securing element and is removable from the recess. The senor can thus be flush with a contact surface with tissue.

Additionally or alternatively the sensor is releasably secured to the device body or the securing element, such as by bonding, for example bonding using a debondable cured adhesive or bonding using an adhesive that forms a bond which fails when a suitable force is applied.

This implant device of the invention is not distinct from other devices of the invention insofar as features of this implant device and features of other devices described herein can be combined in any combination. In particular this implant device can have any of the constructions set out herein and in the claims.

### Brief Description of the Drawings

Embodiments of the invention will be described, by way of example only, with reference to the accompanying drawings. It will be appreciated that some figures are drawn to different scale for ease of illustration.
**Figure 1** shows a fixation device of the invention entering the left atrium via an introducer catheter;
**Figure 2** shows a perspective view of a central spacer, core element or stent element;
**Figure 3** shows a perspective view of a non-moving jaw;
**Figure 4** shows a perspective view of a moveable jaw;
**Figure 5** shows a perspective view of a sliding element;
**Figure 6** shows a fixation device of the invention which is an assembly of the parts shown in **Figures 2 to 5****;**
**Figure 7** shows a section view of the fixation device of **Figure 6****;**
**Figure 8** shows the implant delivery catheter advanced through the introducer catheter and the fixation device of the invention in a capture ready position;
**Figure 9** shows the fixation device advanced below the mitral valve, with the fixation device in a capture ready position;
**Figure 10** shows the fixation device in a capture ready position just below the mitral valve leaflets;
**Figure 11** shows the fixation device in a closed configuration with leaflets captured by the two moveable jaws;
**Figure 12** shows the fixation device in a configuration with one moveable jaw closed and one moveable jaw open;
**Figure 13** shows the fixation device with the two moveable jaws in an inverted configuration and the tissue gripping parts concealed;
**Figure 14** shows the fixation device in a closed configuration with the leaflets recaptured by the two moveable jaws;
**Figure 15** shows the fixation device disconnected from the delivery system and remaining attached to the leaflets in a closed and locked position;
**Figure 16** is a perspective view of the fixation device attached to the catheter in a closed position;
**Figure 17** is a section perspective view of the device attached to the catheter in a closed position;
**Figure 18** shows a section view of the fixation device in a closed but unlocked configuration;
**Figure 19** shows a section view of the fixation device in a closed and locked configuration;
**Figure 20** shows a part-sectional view of a configuration where jaw control rods have been released from the fixation device;
**Figure 21** shows a section view of the fixation element in the closed and locked position with details of a delivery system release mechanism;
**Figure 22** shows a section view of the fixation element in a closed and locked configuration with the delivery system release mechanism slightly proximal of the fixation element;
**Figure 23** shows a perspective view of an embodiment of the fixation element that incorporates rotational gripping features;
**Figure 24** shows a section view (along the line B-B of **Figure 23**) of the fixation element with rotational gripping features in an inverted configuration with the gripping features orientated into a concealed position;
**Figure 25** shows a perspective view of the fixation element in an open position with a number of sensors on the outside face of the non-moving jaw;
**Figure 26** shows a perspective view of the fixation element in an open position with a number of sensors on the outside face of the non-moving jaw;
**Figure 27** shows a perspective view of an embodiment of a fixation element in an open position with sensors location on the cuts on the non-moving jaw;
**Figure 28** shows a perspective view of an embodiment of a fixation element in an open position with sensors removed from the cuts on the non-moving jaw;
**Figure 29** illustrates a sectional view of an movable jaw with schematic representation of sensors below the two gripping elements;
**Figure 30** illustrates a sectional view of a moveable jaw with valve tissue deflecting the gripping elements, the sensors have been activated in this configuration and lights or indicators on the user interface are activated;
**Figure 31** illustrates an embodiment of a fixation device with a different central stent element.
**Figure 32** illustrates an embodiment of the fixation device with an enlarged central stent element.
**Figures 33A and Figure 33B** illustrate different embodiments of the fixation element in situ in a mitral valve from a top view; **Figure 33A** illustrates a smaller central spacer attached to the valve leaflets with a fixation device; **Figure 33B** illustrates an embodiment of the fixation device with a larger central spacer attached to the valve leaflets.
**Figure 33C** illustrates an embodiment of a device with a tricuspid valve attached to the leaflets via the fixation elements.
**Figure 34** shows a sectional view of a device of the invention with a cable that automatically retracts the gripping element(s) and the device is in a gripping configuration.
**Figure 35** shows a sectional view of a device of the invention with a cable that automatically retracts the gripping element(s) and the device is in a passive configuration.

### Detailed Description of the Drawings

The present invention will now be described in relation to the appended drawings.

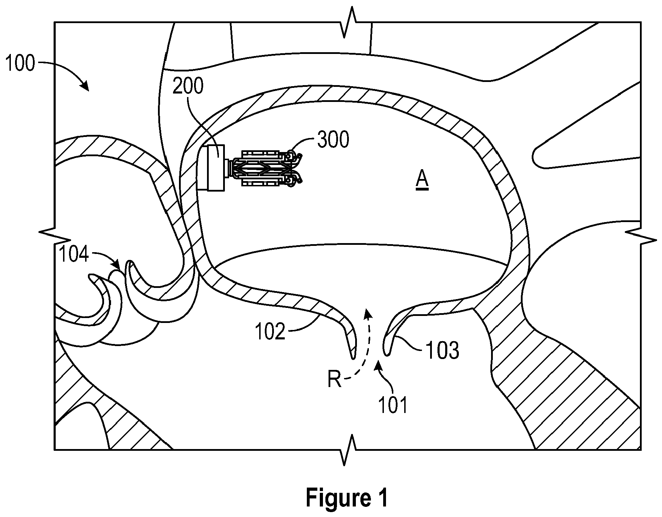

**Figure 1** shows an enlarged sectional view of a part of a heart 100. The heart 100 is illustrated in the systolic phase, just prior to the aortic valve 104 opening. In this phase the mitral valve 101 should be sealed closed, with no gap between its two leaflets 102,103. However there is a gap between the two leaflets 102,103, allowing blood to flow backwards into the left atrium A. This blood flow is indicated by dashed arrow R. In this particular case there is functional mitral valve disease causing regurgitation through the mitral valve 101. As explained above this reduces heart function. Heart failure may result because of this leaky mitral valve

Also illustrated in **Figure 1** is a distal end of an introducer catheter 200 within the left atrium A, the introducer catheter is protruding through the atrial septal wall. The placement of this introducer catheter 200 has been carried out via a precursor step of a transseptal puncture. (Similar to the step that would be conducted as part of a procedure for a left atrial appendage transcatheter procedure.) This introducer catheter 200 acts as a delivery conduit to deliver guide catheters or implant delivery systems transcutaneously. In this position the introducer catheter 200 can be used to deliver interventional tools, implantation devices or catheters into the left atrium/ventricle.

A fixation device 300 of the invention is also shown and will be described in more detail below. It has been advanced through the introducer catheter 200 until it has appeared in the atrium A. It is for delivery to the mitral valve 101 as will be described below.

There are a number of options available for steering an implant such as a fixation device 300 of the invention and any suitable option may be used. A first method includes using an introducer catheter to gain access to the left atrium (as per **Figure 1**). Subsequently a guide catheter that is steerable is introduced through the introducer. The guide catheter is then steered into the correct position and a separate implant delivery system is delivered through the steerable guide catheter and pushed out into the correct position in a target site for example in the heart.

In a second method a steerable introducer catheter along with a steerable guide catheter can be used to place the fixation system in the correct position.

In a third method an introducer catheter is used to gain access to the left atrium (as per **Figure 1**). In this case there is no steerable guide catheter. Instead the implant delivery system is steerable and can be used to deliver the device to the correct position and orientation within the heart.

This latter method is used in the figures.

The fixation device 300 is show in in a compact configuration for travel through the introducer catheter 200/vasculature. The configurations of device 300 will be described in more detail below.

It is to be noted that the fixation device 300 would typically be provided with an external sheath or covering but it is not shown to allow for ease of depiction of a fixation device of the invention. For example it may have a material such as a biocompatible material covering its external parts. The covering could be made of a material such as: PTFE including ePTFE (expanded PTFE), pericardium, PET, polypropylene, Nylon, polyurethane, silicone, polyethylene including UHMWPE (ultrahigh molecular weight polyethylene). The covering's porosity can be varied to encourage fibrous tissue ingrowth or additionally it could be altered to cause certain components to be less permeable and have less or no tissue ingrowth. E.g. the stent element 3100 component could have a covering that is less permeable than the covering that encases the outside surface of the moveable jaws. Preferably, the material that covers the moveable jaws would be suitable to enhance fibrous tissue ingrowth. There are many combinations of coverings that can be used on a clinically implantable device. Different areas could have a cover with different properties.

The attachment of the covering to the fixation device 300 of the invention could be achieved by: tying (for example using sutures), adhesive, welding, fasteners (such as micro rivets), spraying or weaving (for example by weaving a cover onto the fixation device). Different portions of a fixation device of the invention could have covers with different properties and/or be manufactured and/or applied with different processes.

The fixation device 300 of the present invention comprises a number of parts.

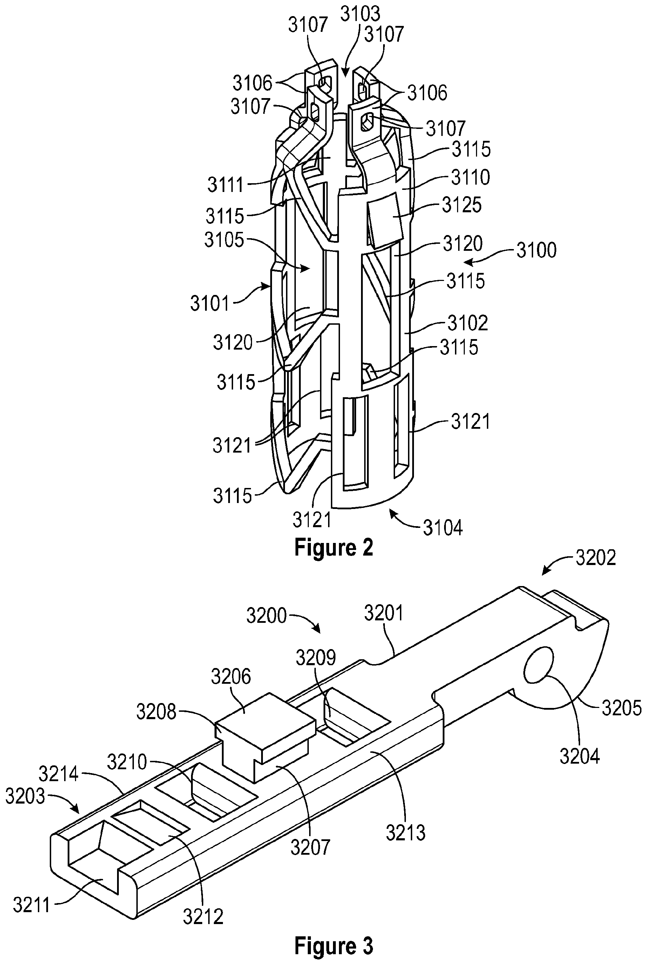

A first part is shown in **Figure 2. Figure 2** shows a perspective view of a central spacer, core element or stent element 3100. This stent element forms the fixation device body. This stent element 3100 is formed by an open frame 3101 formed from interconnecting scaffolding parts 3102. The frame 3101 forms a base frame on which other parts of the fixation device 300 are mounted. The frame 3101 has an open top end 3103 and an open bottom end 3104 generally defining a central lumen 3105. The stent element 3100 thus defines a central space or conduit which runs through the centre of frame 3101. It will be appreciated that a valve can be carried within the frame 3101 and/or the frame can be covered with a non-permeable covering material.

The frame 3101 comprises two opposing and spaced apart side elements 3110 and 3111 which are generally configured to be the same in construction. They are joined by arms 3115. The arms 3115 are generally v-shaped. The arms 3115 are also provided on opposing sides to join the side elements 3110 to each other on both sides. Overall this arrangement forms a stent element 3100 which is generally cylindrical. Furthermore the stent element is deformable. For example the arms 3115 are desirably deformable. For example they may be stretched (e.g. to a more open V-shape) to expand the cross-sectional area of the stent element 300. Side elements 3110 and 3111 may also be deformable for example by stretching.

The arms 3115 have a length of 1.5 mm to 10 mm and similarly the thickness of the arms 3115 could range from 150 microns to 800 microns.

For example if it is desired to change the dimensions and/or shape of the stent element 3100 this can be done as it is expandable. For example a balloon could be used to expand the frame 3101 to keep the same shape but expand the cross sectional area. Additionally or alternatively the shape could be changed. It will be appreciated that a desired shape can be imparted before or after introduction into the body/implantation. In the case of requiring the stent element to be permanently deformed, the stent element material would be an elasto plastic material such as CoCr or Stainless steel. While in the case of requiring temporary deformation, the material could be deformed within its elastic limit or it could be NiTi.

Additionally or alternatively the construction of the stent element 3100 may allow it to be resiliently compressible for example by resilient deformation of the arms 3115. In this way it can be compressed for delivery, for example compressed delivery through a catheter lumen, but expand automatically when deployed, for example when pushed out of the delivery catheter.

At the top end 3103 of the stent element 3100 are struts or brackets 3106 with apertures 3107 defined therein. It will be appreciated that the brackets 3106 are arranged more closely together projecting into the open cross-sectional area defined by the frame 3101. They converge towards a central longitudinal axis of the stent element 3100. In the embodiment four brackets 3106 are shown but two opposing ones would suffice.

The brackets 3106 /apertures 3107 can engage with corresponding features on a delivery system to allow pick up and/or release from the delivery system.

Brackets/apertures may also be provided at the bottom end 3104 if desired, for example to allow further connection to a delivery system such as to provide stability and/or assist in release of the fixation device 300.

The spaced apart side elements 3110 and 3111 each have three longitudinally orientated elongate apertures/slots defined therein.

A first upper slot 3120 is defined respectively therein. These are for receiving and interengaging with formations on a non-moving jaw of a clamp as will be described in more detail below.

A second pair of lower slots 3121 are defined respectively therein. These are for allowing movement of a moving jaw of a clamp of the fixation device 300 of the invention as will be described in more detail below.

The spaced apart side elements 3110 and 3111 each have a protrusion 3125 protruding to the exterior thereof. The respective protrusions 3125 are for interengaging with formations on a non-moving jaw of a clamp of the fixation device of the invention as will be described in more detail below.

The stent element 3100 can be formed of any suitable material. For example it may be cut from a suitable material for example a tube of suitable material. For example it may be laser cut. It may for example be made from nitinol.

**Figure 3** shows a perspective view of a non-moving jaw 3200 of a clamp of the fixation device 300 of the invention. It is formed by an elongate arm 3201 which has a proximal end 3202 and a distal end 3203. A transverse bore 3204 is defined in the non-moving jaw 3200. The transverse bore 3204 allows for pivotal connection of a moving jaw as will be described in more detail below.

The proximal end 3202 defines a cam surface 3205 and this is used in a mechanism to change the position of a gripping element relative to a moveable jaw as will be described in more detail below.

A tab/block 3206 is provided on the arm 3201. It is generally T-shaped in cross-sectional shape having a stem 3207 and a cross-bar 3208. The block 3206 engages within the first upper slot 3120. Respective non-moving jaws 3200 are held onto opposing sides of the stent element 3100 by block 3206. Each stem 3207 extends through a respective upper slot 3120 allowing movement within the slot 3120 but the cross bar 3208 is longer than the width of the slot 3120 and so once in place this holds the non-moving jaw 3200 on the stent element 3100.

There are two further apertures 3209,3210 defined in the arm 3201. These accommodate respective gripping elements in a native valve leaflet gripping configuration of the moveable jaw. They also accommodate gripped tissue. More detail is given below.

There are two additional recesses 3211, 3212 formed in the arm 3201. These are the formations on the non-moving jaw of a clamp of the fixation device mentioned above that engage with a respective protrusion 3125. The first recess 3212 keeps the protrusion 3125 located in a particular position, and consequently this keeps the arm 3201 and stent element 3100 in a relative fixed position prior to a locking action.

The second recess 3211 keeps the protrusion 3125 of the stent element 3100 in a fixed position after a locking action has been completed.

The non-moving jaw 3200 is rounded to prevent sharp edges/damage to tissue. The sides 3213, 3214 of the arm 3201 that interact with the valve tissue are rounded/ filleted to ensure that tissue such as that of a leaflet is not damaged by any sharp or abrupt geometric features.

The arm 3201 can be machined from a block of material, for example by traditional machining methods, or moulded. For example it can be machined by EDM (electrical discharge machining). The arm can also be 3D printed if required. It can be made of any suitable material including metals and alloys such asstainless steel, cobalt, chrome, titanium, nitinol, or a polymer.

Furthermore this component could be manufactured from a tube or flat sheet and manipulated into the required shape (for example shape set NiTi or plastically deformed material) that encompasses all the features that are required in order for it to function.

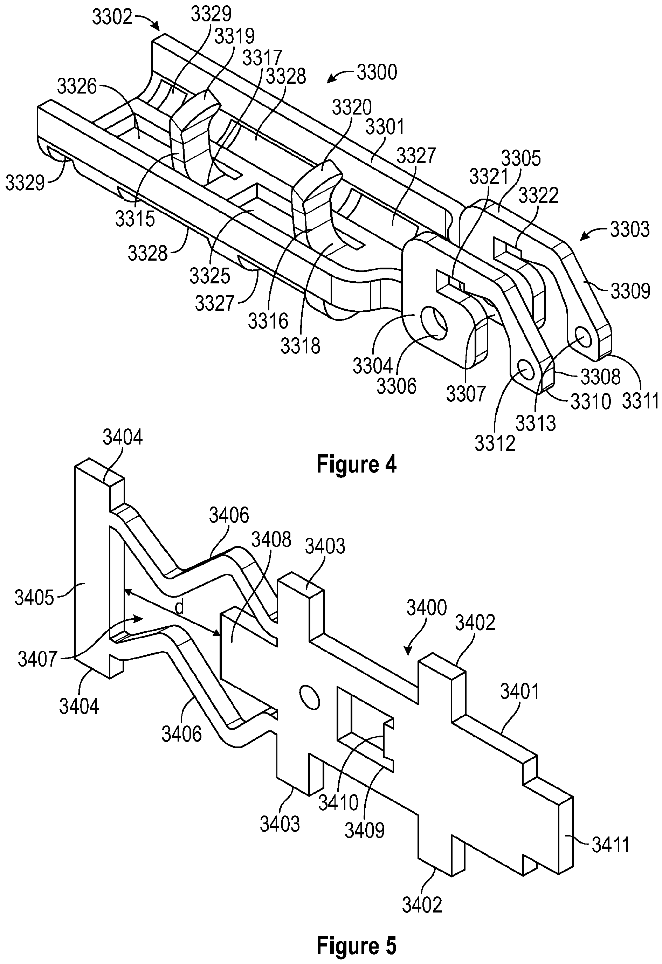

Shown in **Figure 4** is a moveable jaw 3300. **Figure 4** shows a perspective view of a moveable jaw 3300 that together with a non-moving jaw 3200 forms a clamp of the fixation device 300 of the invention. It is formed by an elongate arm 3301 which has a proximal end 3302 and a distal end 3303. On the distal end 3303 is a pair of spaced apart brackets 3304 and 3305. Defined in the respective brackets 3304 and 3305 are respective apertures 3306 and 3307. The apertures 3306 and 3307 are opposed and allow for pivotal attachment of the moveable jaw 3300 to the non-moving jaw 3200. Two opposing curved lever arms 3308 and 3309 extend in a distal direction from the respective brackets 3304,3305. The lever arms 3308 and 3309, have at their respective distal ends 3310 and 3311, respective apertures 3312 and 3313. The apertures 3312 and 3313 are opposed and allow for connection so that the moveable jaw 3330 can be pivoted about that apertures 3306,3307 remotely for example by a delivery device. For example one or more controls such as rods, wires or cables can be connected to the lever arms 3308,3309 and a moving force can then be applied to the lever arms 3308,3309 to move, for example pivot, the moveable jaw 3300 in a desired direction.

On the moveable jaw two gripping elements 3315 and 3316 are provided in the form of resiliently deformable hooks or grips. A respective base 3317 and 3318 of the two gripping elements 3315, 3316 is integrally formed with the arm 3301. A respective top (tissue gripping) part 3319, 3320 of the gripping elements 3315,3316 stands proud of the moveable jaw 3300. This is a native valve leaflet gripping configuration of the moveable jaw 3300. The gripping elements 3315 and 3316 are resiliently deformable and are thus moveable relative to the moveable jaw 3300. As will be described below in the configuration shown in **Figure 4** the gripping elements 3315 and 3316 are arranged for gripping tissue. However the gripping elements 3315 and 3316 may be retracted by a suitable force that causes them to deform e.g. flatten. Once the force is removed they will return (under their own resilient bias) to the configuration shown in **Figure 4****.**

Also formed in the arm 3301 are respective apertures or slots 3325 and 3326. These can accommodate at least a part of the resilient deforming of the respective gripping elements 3315, 3316.

The gripping elements 3315, 3316 can thus be moved between a native valve leaflet gripping configuration of the moveable jaw 3300 where the gripping elements 3315, 3316 are arranged to grip a native valve leaflet 102,103 and a passive configuration of the moveable jaw 3300 where the gripping elements 3315, 3316 are arranged so that the fixation device 300 cannot grip a native valve leaflet 102,103. So the gripping elements 3315, 3316 are used to control the capturing of the valve leaflets 102,103 in a non-traumatic fashion or at least a manner that is minimally traumatic to the tissue. These gripping elements 102,103 are movable from an exposed position to a concealed position. In the exposed position the gripping elements 3315, 3316 engage with the valve leaflets 102,103 to ensure the valve leaflets are captured during the closing cycle of the procedure. In the concealed position the gripping elements 3315, 3316 are in a configuration that will not get entangled with an atrioventricular valve apparatus when the device 300 is being removed, e.g. from the ventricle, or repositioned e.g. within the ventricle. The gripping elements 3315, 3316 are resiliently deformable/elastic in nature. Movement of the gripping elements from the exposed position to the concealed position can be achieved within the elastic range of the material. This enables the gripping elements 3315, 3316 to return to their exposed position once a restraining mechanism is removed.

Also formed in the arm 3301 are a first pair of respective opposed apertures or slots 3327. These can accommodate at least a part of the sliding element 3400 (discussed below). A second pair of respective opposed apertures or slots 3328 also accommodate at least a part of the sliding element 3400. A third pair of respective opposed apertures or slots 3328 also accommodate at least a part of the sliding element 3400.

Notches 3321 and 3322 are provided in the respective curved lever arms 3308,3309 and these form part of a locking mechanism as will be described in more detail below.

The moveable jaw 3300 may be made by any of the methods described above for the non-moving jaw 3200 and/or any of the materials described above for the non-moving jaw 3200.

Shown in **Figure 5** is a sliding element 3400 which forms part of a mechanism for changing the position of the gripping element(s) 3315, 3316 relative to the moveable jaw 3300. In an assembled configuration of the fixation device 300 the sliding element 3400 slides on and relative to the moveable jaw 3300. It is slideable in a first direction across the gripping element(s) 3315, 3316 to move the gripping element(s) 3315, 3316 relative to the moveable jaw 3300. And of course this action can be reversed. This action will be described in more detail below. Furthermore the mechanism for changing the position of the gripping element(s) 3315, 3316 can be used to aid the structural stability of the gripping element(s) 3315, 3316 when they are in the exposed/gripping configuration. In a gripping configuration the control mechanism and in particular the sliding element 3400 provides structural support to the gripping element(s) 3315, 3316 by retaining them in their gripping configuration, for example by pressing against the gripping element(s) 3315, 3316. This acts as a stop to prevent them from returning to the concealed position, especially in the case of an applied external force acting on the gripping element(s) 3315, 3316.

The sliding element 3400 comprises a planar body 3401. The planar body 3401 is dimensioned to be accommodated within and carried by the moving arm 3300. In particular, in an assembled configuration of the device: a first pair of opposing tabs 3402 provided on the planar body 3401 respectively engage within opposing slots 3327 in the moving arm 3300; a second pair of opposing tabs 3403 provided on the planar body 3401 respectively engage within opposing slots 3328 in the moving arm 3300; and a third pair of opposing tabs 3404 provided on the planar body 3401 respectively engage within opposing slots 3329 in the moving arm 3300.

The sliding element 3400 is resiliently deformable. A transverse limb 3405 (which terminates in tabs 3404) part of the planar body 3401 of the sliding element 3400 is joined to the remainder of the planar body 3401 by a part of the planar body 3401 formed by resiliently deformable arms 3406. The arms 3406 are generally zigzag or v-shaped. The arms 3406 are provided on opposing sides of the planar body 3401. Overall this arrangement is resiliently deformable with the distance **d** being variable by resilient compression of arms 3406.

The distance **d** being variable by resilient compression of arms 3406 is achieved as follows. In the assembled configuration of the device mentioned above the a first pair of opposing tabs 3402 provided on the planar body 3401 respectively engage within and are free to move (longitudinally) in opposing slots 3327 in the moving arm 3300 as the tabs 3402 are smaller than the slots 3207. So too the second pair of opposing tabs 3403 provided on the planar body 3401 respectively engage within and are free to move (longitudinally) within opposing slots 3328 in the moving arm 3300 as the tabs 3403 are smaller than the slots 3228. In contrast the third pair of opposing tabs 3404 provided on the planar body 3401 respectively engage within opposing slots 3329 in the moving arm 3300, but because the tabs 3404 fill the slots 3329 the tabs 3404 do not move within the slots 3329. So the sliding action of the sliding element 3400 is achieved by the distance **d** being variable by resilient compression of arms 3406 which is accommodated by the movement of tabs 3402 and tabs 3403 within their respective slots. In this respect it will be appreciated that not all of the sliding element 3400 moves during operation of the mechanism for changing the position of the gripping element(s) 3315, 3316.

An opening 3407 is defined in the planar body 3401 between the arms 3406 and the transverse limb 3405. A stop/abutment block 3408 is provided. Also defined in the planar body is an aperture 3409 in which a stop/abutment block 3410 is provided.

In an assembled configuration of the fixation device 300 of the invention, the two gripping elements 3315 and 3316 protrude the siding element 3400. In particular gripping element 3315 extends through opening 3407 while gripping element 3316 extends through opening 3409. It will be appreciated that the sliding action (which varies distance **d)** will cause the respective blocks/stops 3408 and 3410 to abut the respective gripping elements. In this way the sliding action can be used to retract or withdraw the gripping elements and also allow them to return.

In particular the sliding element sits about the respective bases 3317 and 3318 of the two gripping elements 3315, 3316. Respective top (tissue gripping) part 3319, 3320 of the gripping elements 3315, 3316 stand proud of the sliding element 3400.

Also on the sliding element 3400 is a further abutment end or block 3411. In an assembled configuration of the fixation device 300 of the invention the abutment end 3411 is arranged to abut the cam surface 3205 on the proximal end 3202 of the non-moving jaw 3200 so that together they form part of a mechanism to change the position of the gripping elements 3315, 3316 relative to the moveable jaw 3300.

As the moveable jaw 3300 moves so too does the sliding element 3400 mounted thereon. So as the moveable jaw 3300 moves around a main pivot about apertures 3312, 3313, the abutment end 3411 of the sliding element gets closer to the cam surface 3205 and then contacts the cam surface 3205. As the moveable jaw 3300 is rotated further the arms 3406 of the sliding element 3400 start to resiliently compress and the distance **d** is shortened causing the respective blocks/stops 3408 and 3410 to abut the respective gripping elements and causing them to be progressively depressed and then concealed as the movement of the moveable jaw 3300 continues. Similarly reversing the movement of the moveable jaw 3300 reverses the action of the sliding element 3400. This is achieved as the urging action imparted by the cam surface 3205 is progressively removed and the arms 3406 of the sliding element 3400 start to resiliently return toward their uncompressed state.

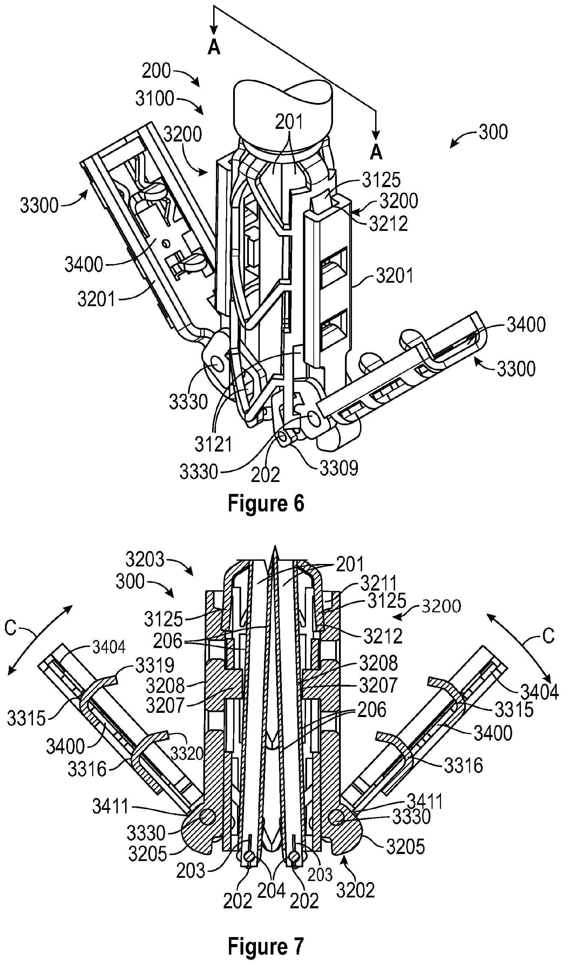

**Figure 6** shows the fixation device 300 of the invention in place on an introducer catheter 200. It will be appreciated that only part of the introducer catheter 200 is shown. **Figure 7** shows a cross-sectional view of the fixation device of **Figure 6** along the direction indicated as **A,A'** in **Figure 6****.**

In **Figures 6** and **7** the stent element 3100, the non-moving jaws 3200, the moveable jaws 3300 and the sliding elements 3400 (those shown in earlier figures) have been assembled to form a fixation device 300 of the invention. One non-moving jaw 3200 together with a moveable jaw 3300 forms a clamp for engaging and holding a native valve leaflet. It will be appreciated that in the embodiment shown there are two such clamps provided by respective pairs of non-moving and moveable jaws 3200, 3300. The clamps are on opposing sides of the stent element 3100.

On respective moveable jaws 3300 are the respective sliding elements 3400.

As explained above the tab/blocks 3206 is provided on the respective arms 3201 are used to attach the non-moving jaw 3200 to the stent element 3100. Also as mentioned above the first recess 3212 keeps the protrusion 3125 located in a particular position, and consequently this keeps the arm 3201 and stent element 3100 in a relative fixed position.

In this embodiment the respective moveable jaw 3300 is pivotally attached to the respective non-moving jaw 3200 by a pin 3330 which runs through apertures 3306,3307 on the moveable jaw 3330 through transverse bore 3204 on the non-moving jaw 3200.

As best seen in **Figure 7** control rods 201 (operated via the introducer catheter 200) extend through stent element 3100 and are attached to lever arms 3308,3309 of the moveable arm 3300 by pins 202. This means the position of the moveable jaws 3300 can be controlled by a user to (pivotally) move the moveable jaws as indicated by arrows **C** in **Figure 7****.** Furthermore the control rods 201 are releasable to allow the fixation device 300 to be released when implanted at a target site. If desired the control rods can be offset relative to each other for example where there is restricted space at the centre of the stent element 3100.

Also, as described previously, and as best seen in **Figure 7****,** abutment end or block 3411 of sliding element 3400, in this assembled configuration of the fixation device 300 of the invention the abutment end 3411, is arranged to abut the cam surface 3205 on the proximal end 3202 of the non-moving jaw 3200 so that together they form part of a mechanism to change the position of the gripping elements 3315, 3316 relative to the moveable jaw 3300. As above this is movement that can be controlled by a user via control rods 201. As described above this action can be used to progressively depress the gripping elements 3315, 3316. Desirably the gripping elements 3315, 3316 are resiliently deformable and sliding element 3400 forms part of a mechanism for changing the position of the gripping elements 3315, 3316 relative to the moveable jaw 3300. It will be appreciated that as the moveable jaw moves the mechanism automatically changes the position of the respective gripping elements relative to the moveable jaw.

The pivot point of the moveable jaw 3300 is specifically arranged to allow the moveable jaw 3300 to rotate around the cam surface 3205 on the non-moving arm 3200. The cam surface 3205 has an outer surface that changes from the top to the bottom of this feature. This allows the distance from fixed points of the moveable jaw 3300 to reduce or increase in distance from a corresponding tangential point on the cam surface 3205. In this way there are in certain positions of the moveable jaw 3300 no force imparted by the sliding element 3400, and in a range of other positions a progressively increasing force. And of course in a reverse (opening) direction a progressively reducing force.

As best seen in **Figure 6** the lever arms 3308, 3309 of the respective moveable jaws 3300 pass through the pair of lower slots 3121 in the stent element 3100. This allows for the freedom of movement of the lever arms 3308, 3309 to impart a wide range of a pivoting action of the moveable jaws 3200. This maximises opening, closing and inversion of the moveable jaws 3300.

The gripping elements 3315, 3316 can be machined from flat sheet or they can have a slightly curved configuration to match a curved configuration that is the geometry of the moveable jaw. The materials used to manufacture this component could be Nitinol or stainless steel or polymers. The machining processes could involve laser cutting or water jet, or EDM or milling machining processes or 3D printing.

The device has been delivered in the closed configuration, not shown, the device could also be delivered in the fully inverted position described below.

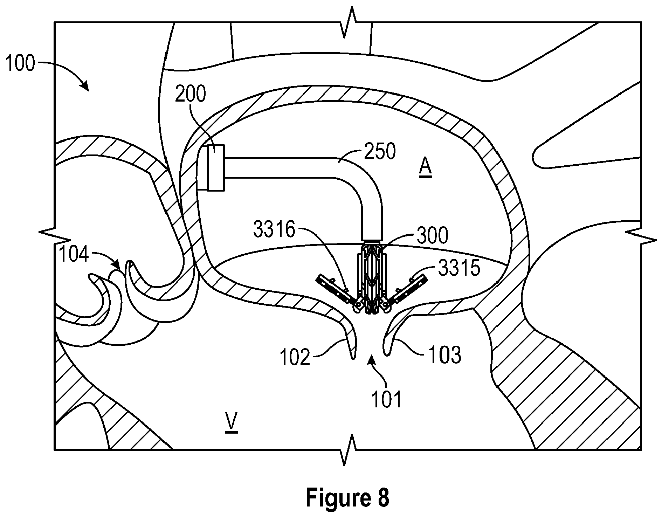

**Figures 8** to **15** show one or more optional implant sequences in the heart 100. In **Figure 8** the implant delivery catheter 250 has been advanced through the introducer catheter 200. The implant delivery system/catheter's configuration has been manipulated or has a pre-set shape to allow it to approach the approximate plane of the mitral valve 101 in a perpendicular manner. The catheter's steering can be achieved by any suitable manner including utilising pull wires, pre-set mandrels or hydraulic actuation of a length of the catheter tubing close to the tip. This section of the catheter may be made up of a multicomponent system (such as a goose-neck system) or a flexible tube that deforms when tension is applied via pull wires or deforms when other mechanical action is applied or deforms when hydraulic pressures are altered in chambers of the catheter's walls.

In **Figure 8** the fixation device 300 is depicted in a capture ready position, or it can be described as being in an open but not inverted position. In this position a number of the gripping elements 3315, 3316 are exposed on the side of the moveable arm 3300 facing the stent element 3100. The gripping elements 3315, 3316 help capture the leaflets 102,103 and keep them captured in the same position while the device is closing. The respective clips or clamps of the device close when a moveable jaw 3300 is closed against the corresponding non-moveable arm 3200. They may also ensure that an appropriate amount of tissue is grasped by the device. They have additional functions, such as keeping the valve leaflets 102,103 grasped while the fixation device 300 remains as an implant in the body. The configuration of the gripping elements 3315, 3316 with tissue tented over them and passing through apertures 3209, 3210 of the non-moving jaw imparts a firm gripping action on the tissue. This type of gripping action, whereby a structural gripping element with heart valve tented over it and passing through a well tolerance hole provides a firm grip to the tissue. When the tissue is in place the tolerance around the tissue and the aperture's sides should not be greater than 1.5mm.

In **Figure 8** the moveable jaws 3200 have been opened by applying a tensile force to the opposed curved lever arms 3308,3309 for example via control rods 201 as described. It is important to note that the fixation device 300 is an implant and it will remain implanted in the body and thus it is releasable from all control/positioning mechanisms. For example, the operator can apply a tension or displacement in the proximal direction, this moves the mechanism proximally which in turn moves the curved lever arms 3308,3309 in a proximal direction which causes the moveable jaw 3300 to open. This mechanism is reversible, for example the mechanism can be advanced distally to cause the curved lever arms 3308,3309 to move distal on the device which in turn causes the moveable jaw 3300 to close. Once the procedure is completed the mechanism can be released from the curved lever arms 3308,3309 allowing the implant to remain in situ in the patient.

The fixation device 300 is orientated with the line of the moveable jaws 3300 being perpendicular to the line of coaptation of the anterior and posterior leaflet 102,103. If the device was used in the tricuspid position, the device clip-line would be orientated perpendicular to the line of coaptation of the two target leaflets. The device's orientation, relative to the line of coaptation, is completed in the atrium A of the heart prior to advancing the implant into the ventricle V of the heart.

As mentioned above the stent element 3100 may be expandable from a configuration with a lower cross-section profile to one which a larger cross-section profile. In **Figure 8** the stent element 3100 has fully expanded. In this particular embodiment the stent element 3100 is made from laser cut nitinol and is expandable. For example it may be constrained e.g. resiliently compressed in the delivery catheter or introducer catheter from expanding to its enlarged shape. However in some instances if the particular condition of mitral regurgitation requires a smaller implant the device may be smaller than the delivery system or fit exactly into the delivery system and in that case the device will not expand as it exits from an introducer or guide catheter.

It will be appreciated that stent element 3100 of the fixation device 300 can be machined from stainless steel or cobalt chrome tubes or sheet or other biocompatible elasto-plastic sheet or tube materials.

In this instance the external contours of the stent element 3100 can be altered by applying a force to the internal surface of the stent element 3100. For example, a balloon or a series of balloons could be inflated within the stent element 3100 to expand the device into a desired shape. The desired shape would be a shape that inhibits a clinically meaningful amount of mitral or tricuspid regurgitation or aids in achieving a more optimal outcome for the procedure. For example, the shape may be manipulated into a configuration that aids the procedure, e.g. the new implant shape could allow easier placement of a second transcatheter edge to edge device. The cross section of this type of implant could be oval, circular, kidney bean shaped, or oblong, triangular, or arrow shaped. This shape would be used to inhibit mitral or tricuspid regurgitation. The desired shape could be achieved by inflating different diameter or different shaped balloons or a number of balloons within the stent element 3100. Deforming the stent element 3100 by inflating balloons within the inside of this component can be easily achieved by threading the balloon or balloons through a central channel of the implant delivery system which may or may not have a guide wire already present.

In the elasto-plastic embodiment of the stent element 3100, the fixation device 300 can be designed to have multiple configurations. This adds significantly to the functionality of the device. For example the device could be designed to expand significantly if a balloon of say 18mm or 20mm or 24mm or 26mm diameter was placed inside it and expanded, the stent element 3100 would achieve a cross section of approximately the diameter of the balloon that had been inflated inside the stent element 3100. This could be achieved by having multiple plastic hinges with long arms of 5mm or 6mm or greater. As the balloon inflates within the stent element 3100 the distance between the moveable jaws 3300 increases as the circumference of the device increases. Any covering on the stent element 3100 needs to account for this increase in perimeter. The design can be covered with a covering that will stretch to account for this increase in the stent element 3100's perimeter, or the covering may have folds that would be released to ensure there is enough material to cover a larger implant. This diameter enlarging procedure could happen during the initial clinical intervention or the stent element 3100 enlarging procedure could happen at a later date. For example, if the patient's mitral regurgitation increased after a number of months or years the operator could locate the proximal or distal orifice of the stent element 3100 and place a guidewire through the device, followed by a balloon over the guidewire. The balloon could be expanded within the stent element 3100 over the guidewire and the stent element 3100's diameter could be increased.

Additionally or alternatively an elastoplastic or self-expanding stent element 3100 can be designed in a manner that if a transcatheter heart valve was delivered into the interior of the stent element 3100 and expanded the stent element 3100 then becomes a structural entity that will hold the delivered transcatheter heart valve in place. Depending on the internal diameter of an elastoplastic stent element 3100 it may or may need to be pre-expanded to allow the transcatheter heart valve to be delivered into the inner section of the stent element 3100. For example if the central spacer was 3mm in diameter it would need to be pre-dilated to 5mm -7mm to accept a transcatheter heart valve of the variety that are available to fix aortic stenosis. The placement of a transcatheter valve in the stent element 3100 could occur during the initial procedure or it could occur after months or years post the initial procedure.

In order for a valve to remain in a desired position within the fixation device 300, an interference fit between the stent element's 3100 structural frame 3101 and a frame of the transcatheter valve will result once the composite structure of the stent element 3100 and transcatheter valve have been combined.

A self-expanding stent element 3100 may have relatively long vertically aligned arms with associated hinges located on its frame. Once a transcatheter valve is implanted or expanded within a self-expanding stent element 3100 these hinge arms may be reconfigured into an orientation where they are more horizontally aligned. The elastic energy stored in the expanded self-expanding stent element 3100 may be used to hold another component or device for example a valve such as an elastoplastic transcatheter heart valve in position or a self-expanding heart valve.

The stent element 3100 of the fixation device 300 could alternatively be expanded mechanically by shortening the device which in turn would cause the stent element 3100 to increase its cross-sectional dimensions.

In another embodiment of the device the stent element 3100 of the device could be manufactured from braided wire and expanded and contracted in a similar manner to the Boston Scientific Lotus^{™} heart valve. Furthermore the device could be locked into the desired diameter once an optimal result was achieved.

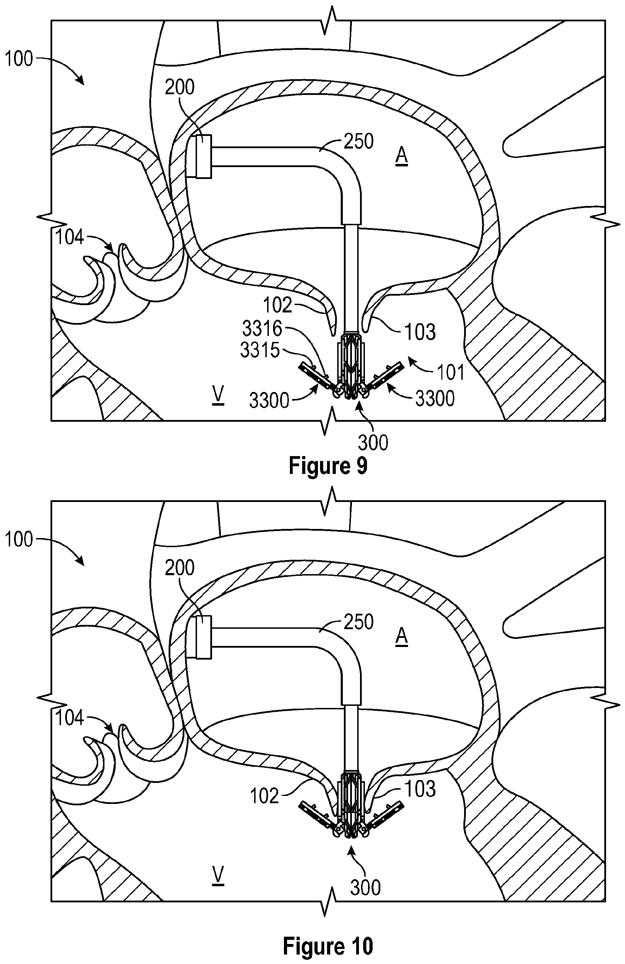

In **Figure 9** the fixation device 300 has been advanced into the left ventricle **V** in an open position with the gripping elements 3315, 3316 exposed. The device has been advanced below the mitral valve leaflets 102,103 and the chordae tendineae. The device has been advanced into the ventricle with the arms perpendicular to the line of coaptation of the two leaflets 102,103 that are the target implantation site for the device of the invention. The device will normally be advanced into the ventricle above the point that is causing the most regurgitation, or the point that the clinical team believes is the best position to deploy the device to eliminate regurgitation as part of a multi-clip deployment strategy.

In **Figure 10** the fixation device 300 has been retracted to just below the valve leaflets 102,103. The two moveable jaws 3300 form gripping arms and they can be closed (for capture of the leaflets) either simultaneously or one at a time. The amount by which they are closed can be controlled by the operator, for example using a handle of the delivery system. The respective gripping elements 3315, 3316 engage leaflets when the respective moveable jaws 3300 are closing and allow continuous engagement of the leaflets 102,103 as the moveable jaws 3300 close. This ensures that the leaflets 102,103 remain between the moveable jaws 3300 and the non-moving jaws 3200 as the device closes.

The moveable jaws 3300 can be moved in tandem or can be moved individually. For example one jaw can be in the closed position with an adequate tissue grasp. The device can then be manipulated towards the un-grasped leaflet and a grasping (moveable jaws 3300 closing manoeuvre) action can be completed on the other leaflet separately to the first action. Similarly the leaflets can be released separately if required.

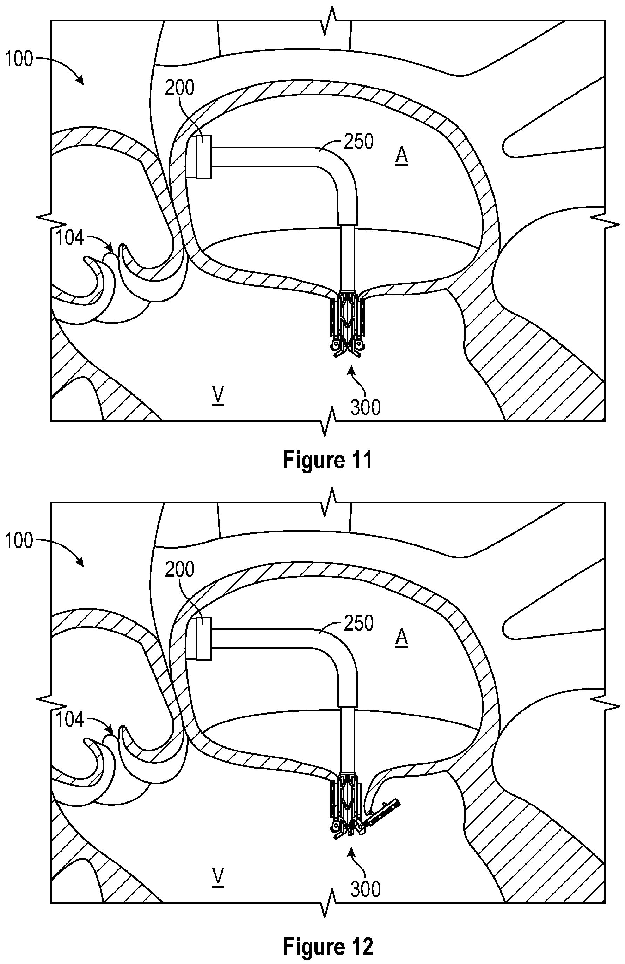

In **Figure 11** both of the moveable jaws 3300 have been closed and the two heart valve leaflets 102,103 have been successfully captured. The moveable jaws 3300 are independently operable so they can be opened (simultaneously or individually) and the device can be repositioned, removed or a single moveable jaw 3300 can be opened and re-closed to try and achieve a better clinical outcome. In this configuration the heart valve leaflet tissue has been pushed through the apertures in the non-movable jaw by the gripping elements of the movable jaw. This configuration of gripping the tissue allows a robust durable solution for this long term implant. Detachment of the device will be minimised with this gripping arrangement.

**Figure 12** shows a typical scenario where a moveable jaw 3300 is open. In this image the device could have been manipulated into this configuration in a number of ways:
In one such scenario, the anterior leaflet could have been captured prior to the posterior leaflet. This could have been an independent closing action that would have been completed by the operator to capture the anterior leaflet in the edge-to-edge device. This leaves the device in the configuration that the moveable jaw 3300 that is targeting the posterior leaflet is still open or inverted.
Prior to closing the moveable jaw 3300 targeting the posterior leaflet, the device's position could be altered to be more approximate to the posterior leaflet. The device could then be manipulated into the position shown just prior to closing the posterior targeting moveable jaw 3300.
The other scenario could have been that both the moveable jaws 3300 were closed in an initial grasping operation. However the operator may not have grasped enough tissue with the posterior moveable jaw 3300 or the operator was not happy with the amount of residual regurgitation and was of the view that repositioning relative to the posterior leaflet was the appropriate step to take. The operator could then retract the control mechanism associated with the posterior leaflet causing it to open. A manipulation of the device could occur or a regrasping of the posterior leaflet could now occur.

To aid capturing the valve leaflet that has not been caught, a second set of arms attached to the non-moving jaws 3200 may be present on the device. Similar in operation and design to the gripper arms on the Mitraclip or Pascal devices, and similar in operation to these arms. This could be especially useful for degenerative mitral or tricuspid valve disease.

In the embodiments shown the non-moving jaws 3200 do not have a second set of gripping arms on them. As described above the non-moving jaws 3200 have apertures 3209,3210 defined in the arm 3201. These accommodate the respective gripping elements 3315,3316 in a native valve leaflet gripping configuration of the moveable jaw. The apertures 3209,3210 also accommodate gripped tissue thus firmly capturing the target valve tissue. The non-moving jaws can also have friction aids on their surface to aid capturing the tissue.

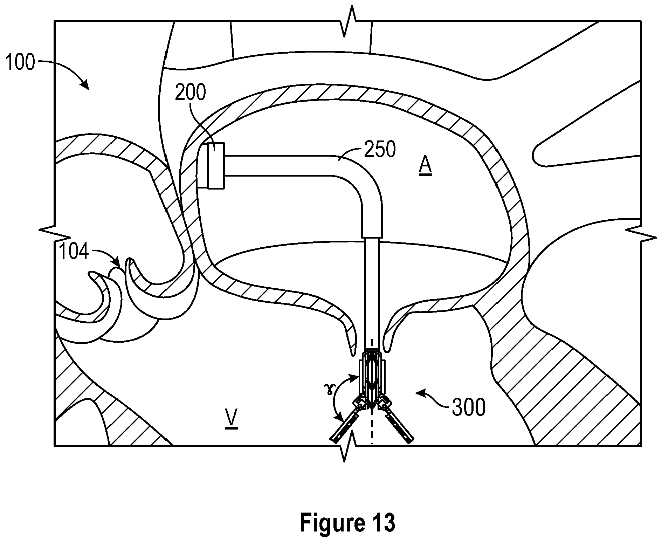

In **Figure 13** the fixation device 300 is shown with both moveable jaws 3300 in an inverted position. By inverted position the inventors mean a position where a moveable jaw 3300 is in a position where a longitudinal axis of the moveable jaws 3300 form an angle of from about 110 to 190 degrees with a central longitudinal axis of the stent element 3100. This refers to the angle on the proximal side of the intersection of those longitudinal axes. This angle is shown as angle **α** in some of the drawings. As above the moveable jaws 3300 are independently operable and can be placed in this position independently of each other. They can be simultaneously placed in this position if desired.

One important aspect of this inverted position is that it provides an atraumatic configuration to the moveable jaws 3300. In particular in this position the gripping elements 3315, 3316 have been retracted. As described above rotation of the moveable jaws 3300 causes the sliding element 3400 to move. In particular during movement to an inverted position the action of the sliding element 3400 against the cam surface 3205 causes the sliding element to move and gradually retract the resiliently deformable gripping elements 3315,3316. So in the inverted position the gripping elements are retracted and are no longer exposed above the moveable jaws 3300.

When both moveable jaws 3300 are in this inverted position they provide an atraumatic configuration to the device, for example when the device is pulled towards the introducer catheter 200 or out of the ventricle V. This is a passive configuration of the device.

One important aspect of this inverted position is in a passive configuration of the device where both moveable jaws 3300 are in this position the device does not have any protruding elements that could catch in chordae or the valve leaflets when it is removed from the ventricle.

This allows an opportunity to withdraw the device and possibly reinsert it into the ventricle.

For example when the device is pulled back into the left atrium A the device can be manipulated to the closed configuration and/or it can pulled back into the delivery catheter. For example it may be configured to be withdrawn into the delivery catheter in this inverted position in particular where both moveable jaws 3300 are in the inverted position.

The inverted configuration of the device may be achieved by retracting the control rods 201.

The inverted position is a preferred position of the moveable jaws to allow the gripping elements to be in a passive configuration. For example the gripping elements may no longer be proud of the moveable jaws. The action may cause the gripping elements to point in a direction which is generally towards the apex of the heart. In this position they will not become entangled with the chordae or the valve leaflets, for example if the device is manipulated within the heart by moving the entire implant proximally.

In **Figure 13** the device 300 is illustrated with the angle **α** between the inner arm and the outer arm approximating 150 degrees. This angle can be increased for example to 180 degrees or it can be increased further to approximately 188 degrees. It will be appreciated that when the moveable jaws 3300 abut the non-moving jaw 3200 the angle **α** will be zero degrees or very small such as less than 10 degrees. It will therefore be appreciated that the range of movement of the moveable jaws is from about zero degrees to at least about 140 degrees, such as at least about 150 degrees, and including up to 190 degrees. The limiting factor to the largest inversion of the moveable jaws is when they contact each other.

In the embodiments mentioned above the implant device 300 is delivered in a closed configuration where the moveable jaws 3300 are closed against the non-moveable jaws 3200 and/or where the angle **α** is zero or less than 10 degrees. Alternatively the device can be delivered in the fully inverted configuration

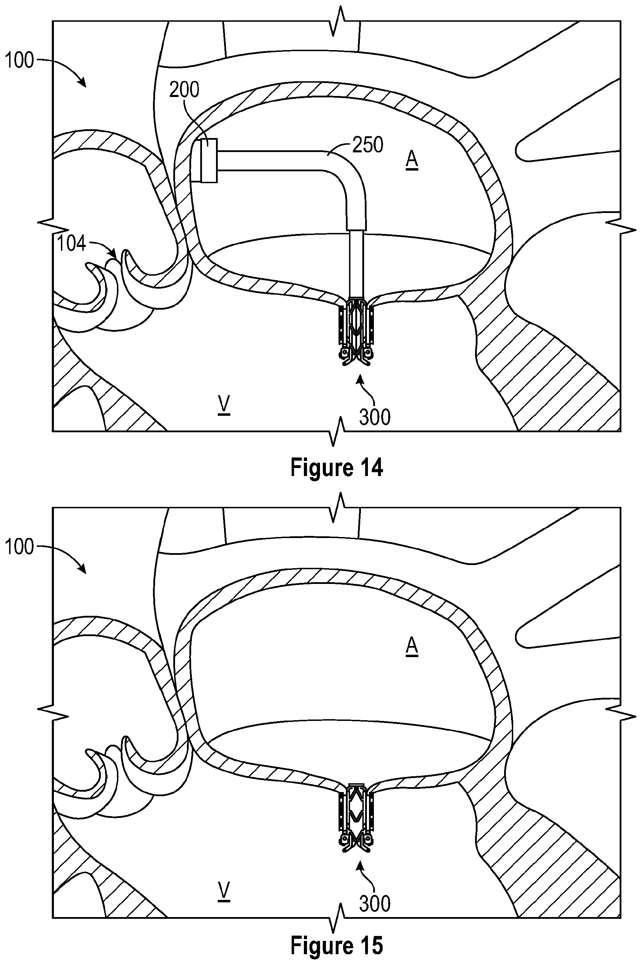

In **Figure 14** the device is still connected to the delivery system (via delivery catheter 250 and introducer catheter 200). The clamps/clips formed between the moveable jaws 3300 and non-moving jaws 3200 clips are closed and have captured valve tissue successfully. A decrease in regurgitation has been achieved.

The stent element 3100 of the implant 300 will be covered and is designed to be non-permeable and will now inhibit or minimise flow through any gap that would have been present as a result of mitral regurgitation in the diseased state. Furthermore the clipping of the leaflets aids the coaptation of the native leaflets and this further enhances the functionality of the native heart valve. The two clamps/clips on either side of the device allow the device to be firmly fixed to the heart valve leaflets.

The device can now be locked into this configuration as the clamps/clips can be locked in the closed position.

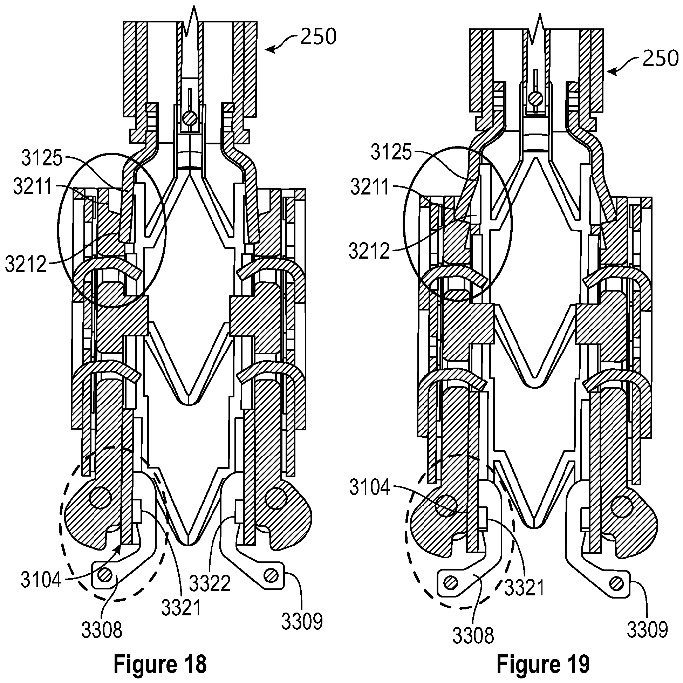

A locking action is also available and is illustrated and discussed below also in relation to **Figures 18** and **19****.** This is achieved by a relative movement between the stent element 3100 and the clamps/clips. This locking mechanism was described above with specific mention of **Figure 6****.** The locking action is achieved by relative movement of the non-moving jaw 3200 to the stent element 3100. This is controlled by a user. Relative movement causes the two additional recesses 3211, 3212 formed in the arm 3201, to move. The protrusion 3125 on the stent element 3100 moves from the first recess 3212 to the second recess 3211. This causes a locking action. The device cannot be reversed from this position.

Furthermore there is a second part to the locking action. The relative movement of the stent element 3100 to the non-moving jaw 3200 causes the notches 3321 and 3322 on the curved lever arms 3308,3309 curved lever arms 3308,3309 to engage with the open bottom end 3104 of the stent element 3100 as will be described blow. This causes the moveable jaw 3300 to be locked so that it can no longer rotate. Additionally the locking is aided by the top surface of the horizontal lower strut associated with slots 3121 becoming abutted against the horizontal surface of notches 3321.

This locking configuration can easily be reversed, by alternating the relative movement of the stent element into the opposite direction than that illustrated in **Figures 18** and **19****.** In this configuration no slots 3121 are required and the protrusion 3125 is orientated in the opposite direction, the moveable jaw will abut against the lower surface of a horizontal element of the stent element.

In **Figure 15** the delivery catheter and the introducer catheter has been removed and the device has been locked in the closed position. So the fixation device 300 is now an implant held in place by its capture of the native valve leaflets.

For example there may be three attachment points between the fixation device 300 and the delivery system/delivery catheter 250 that must be removed to allow the device to remain in situ without any of the delivery system components attached.

In an arrangement of a fixation device 300 of the invention where the moveable jaws 3300 can move independently of each other, each opening/closing mechanism needs to be disconnected from the device separately.

These mechanisms can be connected in the form of one or a combination of the following: a screw thread, a quarter bayonet turn connection, snap-fits, release mechanisms that can be observed on products like the Corevalve^{™} implant release mechanism, release mechanisms such as suture based release mechanisms that can be observed on the gripper control lines on the Mitraclip^{™} device, or release mechanisms such as the release mechanisms associated with the Lotus^{™} aortic valve device, or release mechanisms that can be observed on the Edwards Pascal device, or release mechanisms that are incorporated into atrial septal defect devices.

In one particular embodiment as best seen in **Figure 7** the release mechanism includes pins 202 (discussed above) which are fixed in the apertures 3312 and 3313 of the respective pairs of the lever arms 3308 and 3309. This is connected to the control mechanism via the control rods or tubes 201 with two slits 203 and two circular cuts 204 which are on opposing sides of each of the control rods 201.

The slits 203 run to the end of the control rods 201 and the slits 203 are contiguous with the circular cuts 201. The control rods 201 can be machined from nitinol and the end of the tube is set in an open-jaw configuration.

The pins 202 passes through the two circular cuts 204. The pins 202 are retained in the circular cuts 204 by compressing opposing sides of the control rods 201.

The compression of the two sides of the control rods 201 is achieved by passing a restraining tube 206 over the outside of control rod 201.

To release this control mechanism from the fixation device 300 the restraining tube 206 is moved proximally relative to the control rod 201. This proximal movement of the restraining tube 206 causes the control rods 201 to resiliently expand. This opens the slits 203 and allows the pins 202 to be released. Both the control rods 201 and their respective restraining tubes 206 can be moved proximal and removed from the implanted device. This operation can be repeated for the other clip. And of course this release can be sequential or simultaneous.

The stent element 3100 may have its own release mechanism which could be one of the above mentioned release mechanisms or it could be a combination of release mechanisms that may or may not include mechanisms mentioned above or additionally a threaded mechanism that is observed on the Edwards^{™} Pascal^{™} device or the Abbott^{™} Mitraclip^{™} device.

The mechanisms employed for releasing transcatheter devices are well understood and have been used on many devices in the structural heart space including aortic valve replacements, left atrial appendage devices, atrial septal defect devices and transcatheter mitral valve repair and replacement devices.

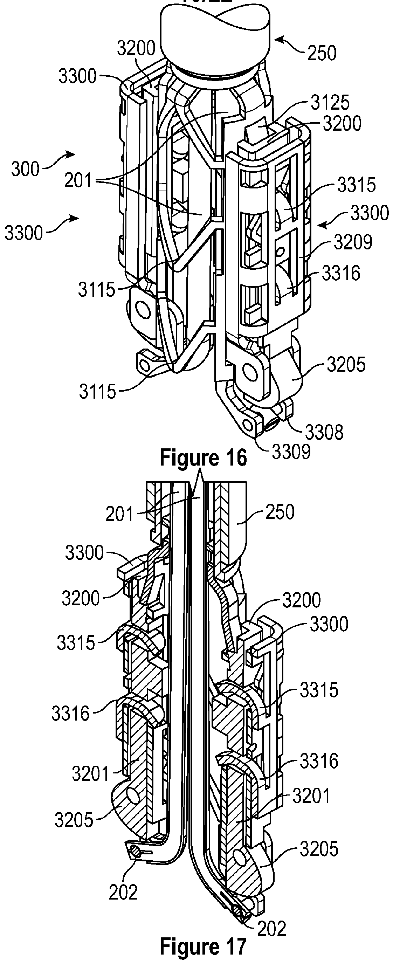

**Figure 16** and **Figure 17** show the fixation device 300 in a closed or capture position. **Figure 16** shows a perspective view while **Figure 17** is a part sectional view. The respective clamps/clips formed by the respective pairs of jaws 3200,3300 lie substantially parallel with a longitudinal axis of the stent element 3100.

The clamps and in particular the moveable jaws 3300 thereof do not have to lie parallel with the longitudinal axis of the stent element 3100 when they are in the closed position. Depending on the amount of tissue being captured etc. the closed position may result in the moveable jaws 3300 being open by a number of degrees. For example the angle **α** could be less than 10 degrees.

It will be appreciated that in the closed position is a native valve leaflet gripping configuration of the moveable jaw(s) 3300.

As discussed above, in a clinical procedure, movement of the control mechanisms will cause the clamps/clips to close and the valve leaflet tissue will be caught in between the stent element 3100 and the moveable jaw 3300. The gripping elements 3315,3316 will aid holding the tissue in this location as well as the compressive force applied by the arm 3201 of the moveable jaw 3300. Furthermore as the gripping elements 3315,3316 pass through non-moving jaw 3200, they pull the tissue through the apertures 3209,3210 in the moveable jaw 3300. Furthermore the gripping elements 3315,3316 may also pass through the slots or holes 3120.3121 in the central spacer 3100. It will be appreciated that the non-moving jaw 3200 could be recessed instead of having apertures defined therein. In such an arrangement the gripping elements 3315,3316 and or any tissue captured by them will sit within the recesses.

If desired the gripping elements 3315,3316 may be adapted to pierce, rather than just grip, the tissue. For example the tips of the gripping elements that engage tissue can have different configuration ranging from blunt to piercing and configurations in between, for example a short serration, where the gripping elements can catch the tissue but not pierce the tissue.

It will be noted especially from **Figure 17** that the control mechanism is in a curved configuration when the device is closed. Such a configuration requires the control mechanism to be flexible yet have sufficient compressive/tensile strength to impart a rotation to the moveable jaws 3300. In particular the control rods 201 and their respective restraining tube 206 should be sufficiently flexible. This dual requirement for strength and flexibility can be achieved with suitable materials including suitable tubes such as hypotubes which are optionally laser cut, polymeric tubes such as braided polymeric tubes or other similar arrangements including combinations of different tube types.

**Figures 18** and **19** show how a locking action is achieved. As discussed above the locking action is achieved by relative movement of the non-moving jaw 3200 to the stent element 3100. This is controlled by a user. Relative movement causes the two additional recesses 3211, 3212 formed in the arm 3201, to move. The protrusion 3125 on the stent element 3100 moves from the first recess 3212 to the second recess 3211. This causes a locking action. The device cannot be reversed from this position. This movement is shown in the sequence/comparison of **Figure 18** to **Figure 19** and is highlighted in each drawing by an ellipse shown in unbroken line.

The relative movement of the stent element 3100 to the non-moving jaw 3200 also causes the notches 3321 and 3322 on the curved lever arms 3308,3309 curved lever arms 3308,3309 to engage with the open bottom end 3104 of the stent element 3100. This causes the respective moveable jaws 3300 to be locked so that they can no longer rotate. This movement is shown in the sequence/comparison of **Figure 18** to **Figure 19** and is highlighted in each drawing by an ellipse shown in broken line.

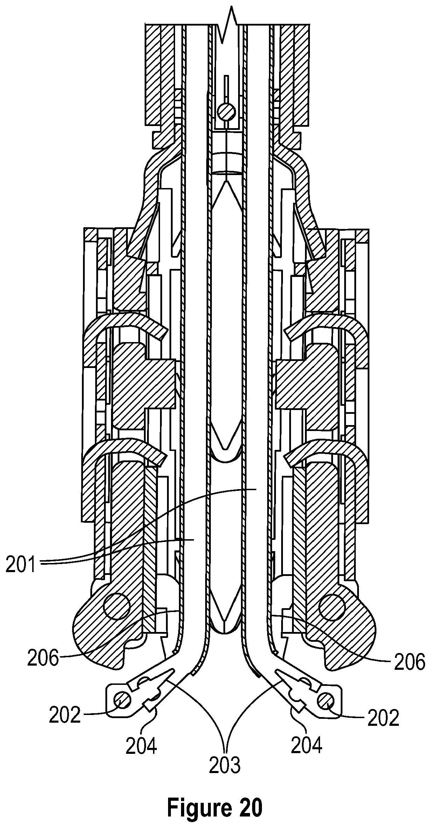

**Figure 20** shows a part-sectional view of a configuration where the control rods 201 have released the fixation device 300. As discussed above the pins 202 are retained in the circular cuts 204 by compressing opposing sides of the control rods 201. To release this control mechanism from the fixation device 300 the restraining tube 206 has been moved proximally relative to the control rod 201. This proximal movement of the restraining tube 206 causes the control rods 201 to resiliently expand. This opens the slits 203 and allows the pins 202 to be released. **Figure 20** shows the configuration where this has already been done and both the control rods 201 and their respective restraining tubes 206 can be moved proximally and removed from the implanted device. This release can be sequential or simultaneous for both clips/clamps.

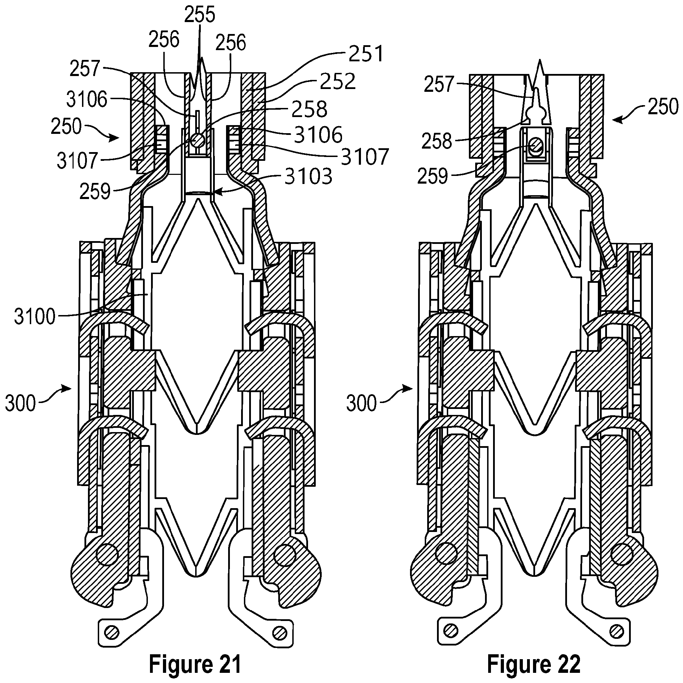

**Figures 21** and **22** show more detail on how the fixation device 300 is released from the delivery catheter 250. The delivery catheter 250 includes an inner tube 251 and an outer tube 252. As shown the inner tube 251 engages with two of the struts or brackets 3106 at the top end of open frame 3101 of the fixation device 300. In particular formations on the inner tube 251 engage within apertures 3107 in the struts or brackets 3106. Within the delivery catheter 250 is an attachment mechanism which is very similar in construction to the attachment mechanism for the control rods 201 to the moveable jaw 3300. An inner tube 255 is provided with a slit 257 and a circular cut 258. The slit and cut are resiliently biased toward an open position. A restraining tube 256 holds the slit and circular cut closed as shown in the configuration of **Figure 21****.** A pin 258 runs between two of the struts or brackets 3106 not held by the inner tube 251. This pin 258 is retained within the circular cut in 255 so that the fixation device is attached to the inner tube 255 and held in place by outer restraining tube 256. As shown in in **Figure 22** as the outer restraining tube is moved proximally this allows slit 257 and circular cut 258 to open to release the top end 3103 of the stent element 3100. This is a desirable arrangement for the present invention but of course other attachment/release mechanisms could be employed.

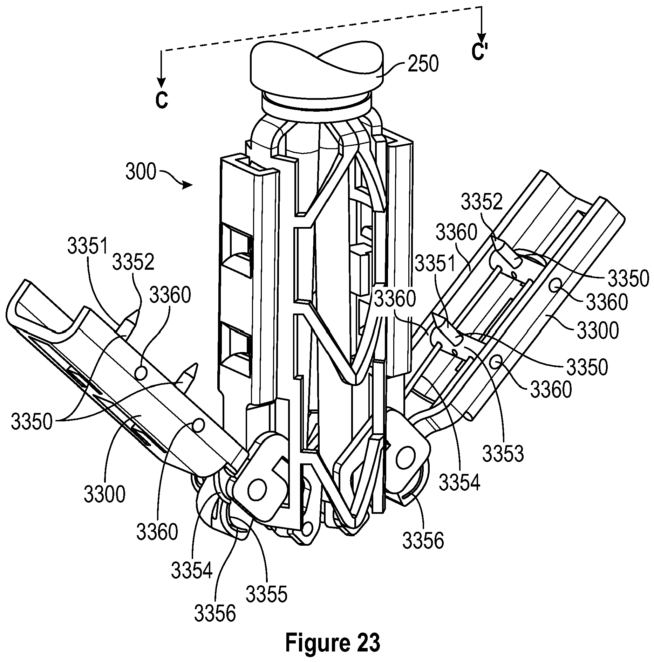

In the embodiment shown in **Figure 23** the gripping elements 3350 are not an integral part of the moveable jaw, they are separate components connected to the moveable jaw. In particular the gripping elements 3350 each comprise a pin 3351 with a pointed tip 3352. Each pin 3351 is mounted perpendicularly to the axis of a pivot axle 3353. Each pivot axle 3353 is mounted to the moveable jaw 3300. In particular the pivot axle is mounted transversely of a longitudinal axis of the moveable jaw 3300. Mounting of each pivot axle 3353 is by way of pairs of opposed retaining apertures 3360 defined in opposing sides of the moveable jaw 3300. In this arrangement the pivot axles 3353 and thus the gripping elements 3350 can be raised and lowered. This allows the gripping elements 3350 to be moveable relative to the moveable jaw 3300 between a native valve leaflet gripping configuration of the moveable jaw where the gripping element(s) 3350 is arranged to grip a native valve leaflet and a passive configuration of the moveable jaw where the gripping element(s) 3350 is arranged so that it cannot grip a native valve leaflet. In **Figure 23** the position/orientation of the gripping elements 3350 are controlled by a tensile member rather than a sliding element. In particular the pivoting action of the gripping elements 3350 is controlled by a tensile member. Optionally and desirably the gripping elements are biased towards the native valve leaflet gripping configuration. They may be biased toward the passive configuration also. In **Figure 23** the gripping elements are shown in a position where the pointed tips 3352 thereof stand proud of the gripping arms 3300. This is a native valve leaflet gripping configuration. Other than the tie line and the gripping elements the device shown is very similar in construction to that of earlier figures.

The orientation of the gripping elements 3350 can be altered by applying or releasing tension in the tensile member. In the case of the embodiment the tensile member is a tie line or cable 3354. As can be best seen on the right hand side of **Figure 23** the tie line 3354 runs (twice) through each of gripping elements 3350 in a loop so that the gripping elements 3350 on a given moveable jaw 3300 move in unison.

The tie line 3354 also runs to a fixed point so that as the gripping jaw 3300 is moved tension in the tie line is increased or reduced. As can be best seen on the left hand side of **Figure 23****,** the tie line 3354 has an anchor point 3355 which is on the non-moving jaw 3200 and in particular within a curved slot 3356. When the moveable jaw 3300 is opened or closed the distance between the point on a gripping element 3350 and an interacting point on the non-moving jaw 3200 changes and this has the effect of changing the amount of tension within the tie line 3314. The tie line can be adapted as desired for example to configurations whereby there is no tension in it and it is slack, to a configuration where for a certain opening arc of the moveable jaw there is a pre-set tension within the tie line. Or for example the tie line has a maximum tension for a certain point of closure.

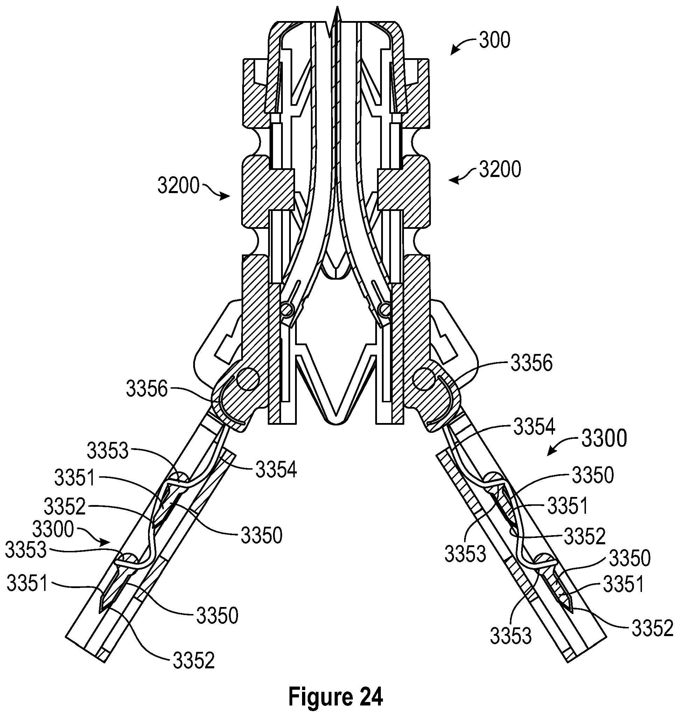

For example in a capture ready position the gripping elements 3350 are erected based on the amount of tension in the tie line 3354 and are retracted in a passive configuration of the device. As described above in relation to the embodiment which includes a sliding element there are desired orientations to have the gripping elements in a passive configuration. For example the passive configuration is desirable as the device opens and moves towards an inverted configuration. A capture ready position is desirable as the device closes. For example **Figure 24** shows (in cross-sectional view) a passive inverted configuration of the device of **Figure 23****.**

It will be appreciated that the tie line/tensile member can be arranged so that the gripping elements are in their exposed configuration when the tensile member is slack. Or the tensile member and gripping elements may be arranged in such a manner that when the tensile member is taut the gripping elements are raised/exposed. The operation of the gripping elements can be easily changed by routing the tensile member in a different manner to the route that is shown in this figure so that the tension is applied at a different relative position of the moveable jaw. In essence the direction of tensile force applied to the gripping members can be reversed.

Furthermore there may be resilient bias of the gripping element(s) and/or the tensile member(s) that causes the gripping element(s) either to pop up or lie flat when the tension is released from the tensile member(s).

In the embodiment shown in **Figures 23** and **24** gripping elements that rotate within the moveable jaws are present. These gripping elements are designed to be exposed or concealed. In this embodiment the gripping elements are simple circular rotational elements. As the tension member applies more tension these rotational elements are exposed. If the tension is released in the tension member the gripping elements are free to rotate into a concealed position pointing towards the apex of the heart especially if the device assumes an inverted position.

In other embodiments flat gripping elements can lie flat with a direction of the gripping element that engages with the valve tissue pointing towards the unconstrained end of the moveable jaw. These flat elements may have tabs that engage in curved slots in the wall of the moveable jaw. The tension member will pass through the gripping elements. As the tension member becomes taut via a movement of the moveable jaw, the flat gripping elements rise and are exposed and can engage the valve tissue.

In the embodiment of **Figure 23** and **Figure 24** the distance from the curved slot 3356 to the distance the tensile member interacts with the gripping element increases as the moveable jaw closes or decreases as the moveable jaw opens. This change in distance will cause tension to be imparted to the tensile member as the moveable jaw closes.

By selecting an appropriately positioned and shaped curved slot, the distance can be set by the curved slot to be constant/fixed for the last portion of the closing action of the moveable jaw.

For example, there may be the same distance between the slot and the gripping element for the last part of the closing action for example the last 70 degrees. This means there is no additional tension applied to the tensile member within that range of movement. This in turn means that the gripping element can remain in a fixed position for the last portion of the arc that is swept out as the moveable jaw closes. While for the remaining amount of the clip closing cycle, e.g. from an inverted position to the last part of the closing action for example the 70 degree closing position the distance between the slot and the gripping element decreases which in turn causes the tensile member to move the gripping elements from the passive configuration to a gripping configuration for example where they are aligned with a face of the moveable jaw.

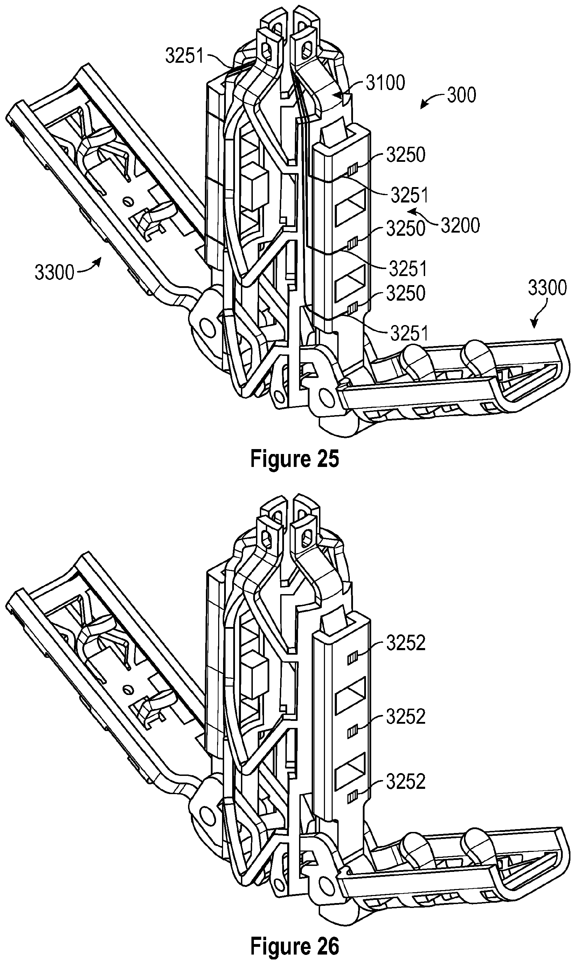

**Figure 25** shows a perspective view of a fixation device 300 which is very similar in construction to the embodiments of **Figure 22** and earlier figures. This aspect of the invention applies to all embodiments. Sensors 3250 have been provided. One or more such sensors can be provided at any suitable location in the fixation device. The purpose of the sensor(s) is to provide an indication/feedback to a user as to the capture of tissue by the device. As mentioned above the sensor(s) may be at any location on the device provided they provide an indication/feedback in relation to tissue captured by the device. They may be provided on one or both of the non-moving jaws 3200 as shown in **Figure 25****.** Electrical connectivity is provided to the device by insulated wiring 3251. The wiring runs up the device and can run back through any delivery/introducer catheter. In **Figure 25** because of the orientation of the device the sensors cannot be seen on the non-moving jaw 3200 on the left hand side of the device but they are present as are indicated by the wiring 3251 on the left hand side of the device. The wiring can thus be connected to a suitable controller which can power the sensors and/or receive feedback from the sensors. An important aspect of the present invention is that the sensors 3250 can be removed from the fixation device 300 following implantation. As shown in **Figure 26** the sensors 3250 have been removed from respective receiving apertures 3252 in non-moving jaw 3200. Also all associated wiring 3251 has been removed. The removal has occurred through any delivery/introducer catheter. (In order to illustrate the removal of the sensors 3250/wiring 3251 the moveable jaws 3300 are shown in an open position whereas they would be in a closed position following capture of tissue.) The device is configured so that the sensors/wiring are removable (via a control mechanism) after tissue capture and when the device is in a closed position.

The sensors utilised do not perform any function post the delivery phase. These sensors can thus be designed to simply be removed from recesses or channels and be removed by applying a proximal displacement for example utilising the connecting elements on the user interface. Additionally or alternatively to being located in apertures the sensors can be detachably attached, for example weakly bonded e.g. using a suitable adhesive, to the fixation device and this attachment/bond can be broken by proximal movement. This may be achieved by movement of the sensor connection wires and/or structural elements of the device and/or movement of controls.

The function of the sensors is to provide feedback to the user as to whether sufficient tissue has been captured within the clip/ clamp formed by the two jaws 3200,3300 when the device is in the closed configuration. When the device closes and tissue is caught at the location of the sensor a feedback system, for example one located on a handle of a control mechanism, lets the user know the degree, amount of tissue that has been captured. This is especially useful as this type of system removes uncertainty during the procedure.

Any sensor capable of providing feedback on the amount of tissue captured is desirable. For example the sensors could be pressure sensors, infrared based sensors, laser type sensors, ultrasound sensors or any combination thereof. For example if tissue, such as that of a leaflet, is pressed against the sensor the sensor may alter its electrical resistance and the feedback system can then indicate, for example light up a light illustrating that the device has caught tissue at this location.

It is desirable to provide a plurality of, for example a series of at least three, sensors along each clip/clamp of the device which can individually indicate whether or not tissue has been sufficiently captured at each location. Accordingly a user can get feedback as to successful capture across a range of locations of a given clip/clamp. And of course, and as described above, if the user is not satisfied with tissue capture in one or both clamps/clips the user can independently or simultaneously release the captured tissue from the clips/clamps and then recapture for better location of the device. Once satisfied the user can lock the device in place as described above and then remove the sensors and any control mechanisms attached to the device to leave he device implanted without sensors present.

As mentioned above any suitable sensor can be utilised. One alternative to sensors that require electrical/electronics to provide feedback is to provide a visual indication. For example a visual indicator can be provided of successful capture of tissue. This may be viewed by a user using an imaging technique such as fluoroscopy. So rather than being sensors that give a direct feedback to the user via changes in an electric circuit the sensors may give a visual indication, for example may be radiopaque. For example the visual indicator may move/deform if tissue is captured. For example a radiopaque sensor may assumes a different geometry when tissue is captured. For example one sensor or an array of sensors could be present on the device for example on one or both non-moving jaws or one or both moveable arms or any combination of same. These sensors can be in a geometry that protrudes from non-moveable jaw and/or the moveable jaw and can easily bend or deform. These sensors or feedback mechanisms will be observed on fluoroscopy as being protruding if no tissue has been caught in the clip/clamp. While if the clip/clamp captures tissue the sensors will deform into a new shape. So the operator can observe a deformed sensor and will know if they have captured any or sufficient tissue successfully. And as above the user can then release and recapture to better position the device. Radiopaque sensors can be constructed of gold, platinum or another heavy metal or alloy combination and they will show up on fluoroscopy.

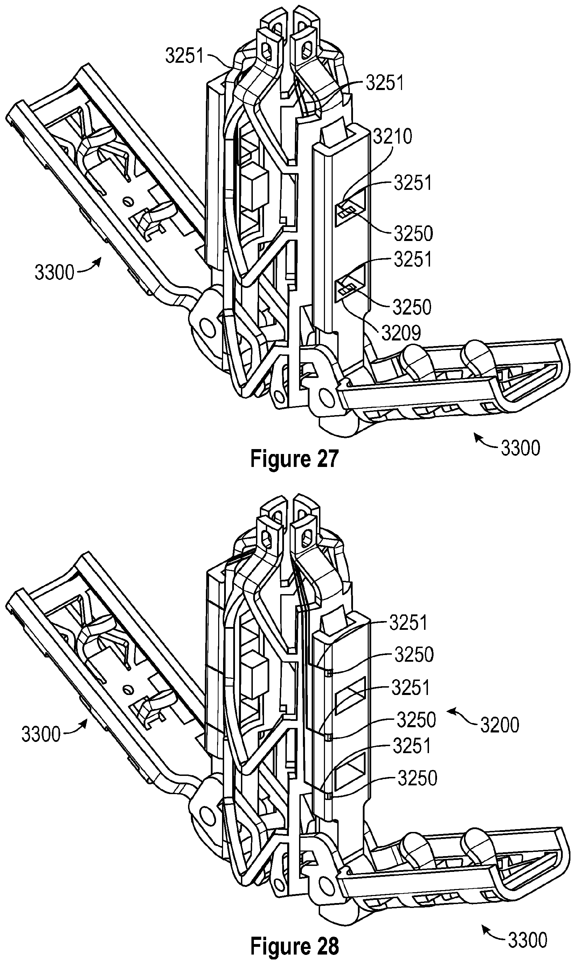

**Figure 27** shows an alternative/additional positioning for sensors 3250 (and consequently their wiring 3251). In this embodiment they have been positioned within the apertures 3209/3210 in the non-moving jaw 3200. In this arrangement the sensors may detect whether tissue is present within the apertures 3209/3210 which in turn is an indication of how successful capture of tissue has been. For example, if the gripping arm 3300 has not caught any tissue the sensor(s) may indicate that no tissue is captured e.g. by lighting a red light. If tissue has been caught the sensor will indicate same and for example a green light may be lit. And as discussed above as there are a plurality of sensors they can independently indicate if tissue is captured at each location thus giving the user a good indication of the capture of tissue at a number of locations. In this case the sensors may operate on the basis of metal-metal contact. When tissue is present there is no metal to metal contact between the two arms 3200/3300. When tissue is not present there may be metal to metal contact. These sensors are removable also as described above. Additionally or alternatively other sensors such as mechanical radiopaque sensors can be used in this location too.

**Figure 28** shows an alternative/additional positioning for sensors 3250 (and consequently their wiring 3251). In this embodiment they have been positioned on the sides of the non-moving jaw 3200. In this arrangement the sensors may detect whether tissue is present between the non-moving jaw 3200 and the moveable jaw 3300 which in turn is an indication of how successful capture of tissue has been. Again if desired the sensors are removable.

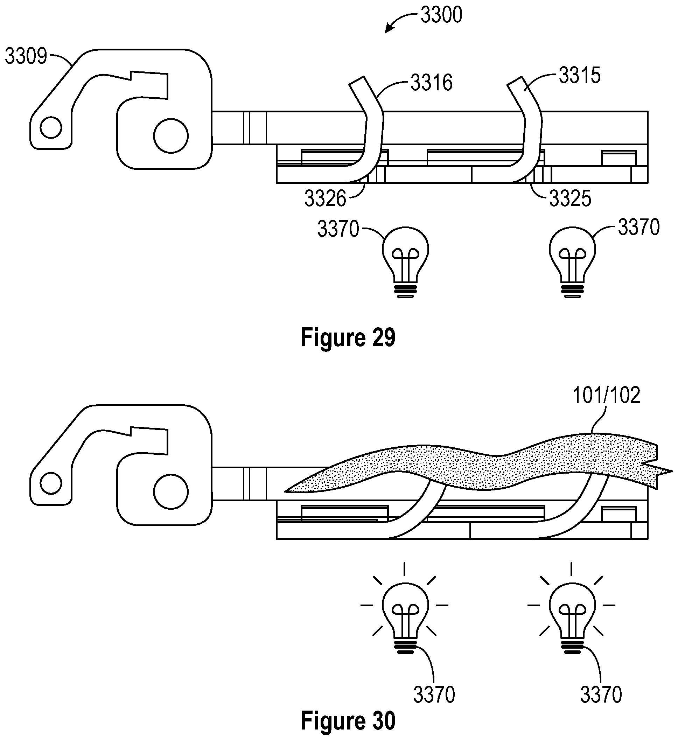

**Figures 29** and **30** show an alternative/additional mechanism for detecting tissue capture that may be utilised in addition to, or instead of, sensors. In this embodiment the gripping element(s) and a contact are arranged so that when the clip/clamp grips tissue such as a native valve leaflet, deformation of the gripping element(s) occurs causing the gripping element to move against the contact. This allows for detection. In this case the detection of gripped tissue is by the moveable arm 3300 but of course this arrangement can be positioned elsewhere on the device.

In particular gripping elements 3315 and 3316 are arranged on the moveable jaw 3300 as described above. As tissue such as a valve leaflet 101/102 is gripped the gripping elements 3315 and 3316 deform. If sufficient tissue is captured the gripping elements 3315 and 3316 deform sufficiently. The deformation may be sufficient to make a physical contact of the gripping element to a contact which may be a sensor. Additionally or alternatively a sensor for the deformation of the gripping element such as a strain gauge may be utilised. In any event when sufficient deformation of the gripping elements is detected the user will receive an indication. For example when the contact is made, lights 3370 may light up. The lights 3370 will of course be at the user interface. **Figure 30** shows a configuration where sufficient capture of leaflets has occurred and the gripping elements have been deformed sufficiently to cause contact and light up lights 3370.

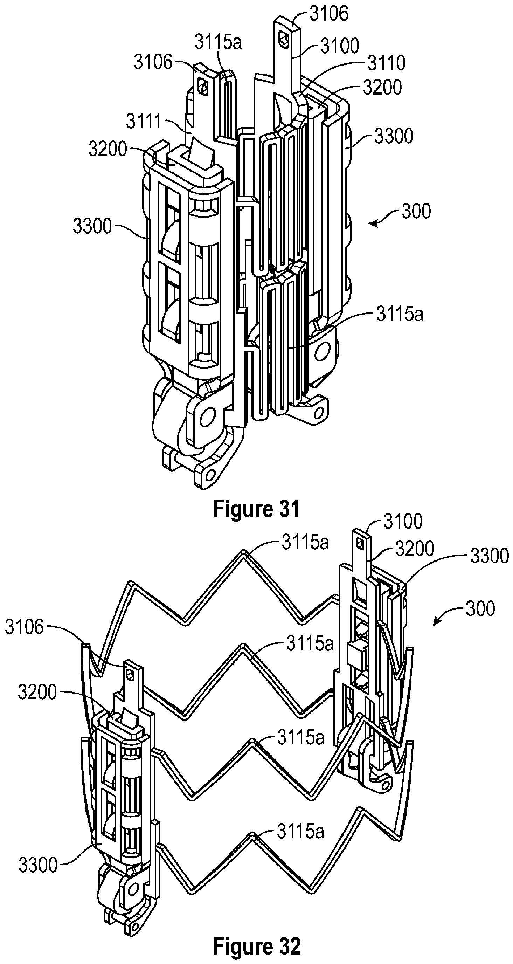

**Figure 31** shows a perspective view of a fixation device 300. The device is the same as in previous figures save that the central spacer, core element or stent element 3100 is changed. In this embodiment the stent element 3100 has side elements 3110,3111 which are the same as in previous figures. However the side elements are now joined by arms 3115a which are folded back and forth in an accordion type arrangement and which allow a greater degree of expansion of the stent element 3100 than arms 311 utilised in previous figures. **Figure 32** shows an expanded configuration where a balloon or other expansion means has been used to increase the diameter of the stent element by expanding out the arms 3115a. Alternatively the arms 3115a could be resiliently biased towards the expanded position of **Figure 32** but for delivery is constrained to the unexpanded configuration of **Figure 31****.** Once the constraint is released the fixation device then automatically expands to its expanded configuration.

The unexpanded configuration could be 3mm in diameter or 5mm in diameter or 10mm in diameter or a diameter within this range or close to this range. The expanded diameter would be larger than this and could be 15mm or 20mm or larger or smaller.

As seen the struts/brackets 3106 may be of a different configuration in such an arrangement as compared to earlier figures.

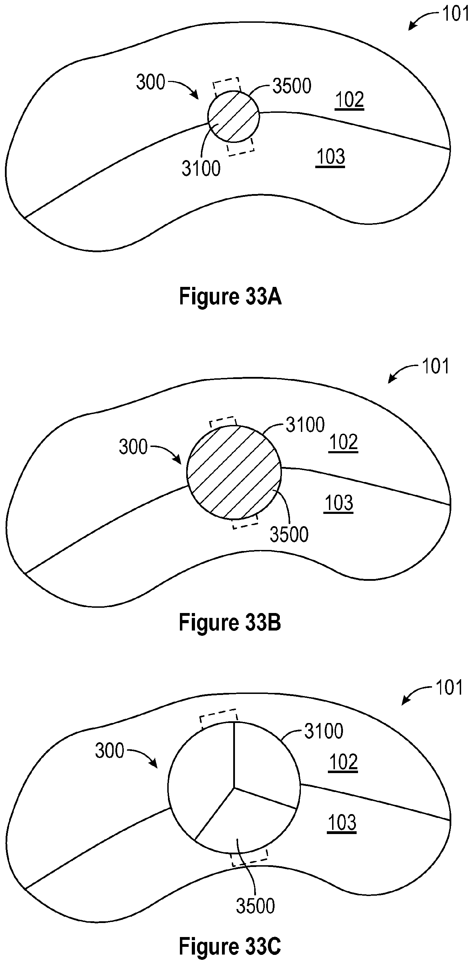

**Figures 33A to 33C** show a schematic representation of a fixation device 300 of the invention implanted in a mitral valve 101 and with leaflets 102/103 captured. The device is expandable. In **Figure 33A** the device is shown implanted in an unexpanded configuration. (The broken lines indicate the clips/clamps.) A valve 3500 is present in the stent body 3100. Initially it is folded/unexpanded. In **Figure 33B** the device 300 is expanded. As before this can be by resilient expansion or by being expanded by an expanding means such as a balloon. This expansion allows the device to be altered at a later date giving the patient access to an easy percutaneous solution that can avoid the need for open heart surgery at a later date. In this instance the device is made from an elastoplastic material. An important aspect of this feature is that it provides the user with an option to alter the implant diameter either before it is attached to the leaflets or once it is attached to the valve leaflets. For example when the device is delivered and regurgitation is still present the user can dilate the implant to inhibit regurgitation while the implant is attached to the valve leaflets. Additionally, if the user has checked with different balloon sizes prior to delivering and fixing the devices to the leaflets the user can preload the device with the correct balloon size to enlarge the device and minimise regurgitation. A deformable central stent element is useful to inhibit regurgitation or to minimise regurgitation, the deformation could be associated with a restrained self- expanding stent element or an elasto plastic stent element.

In **Figure 33C** the device is illustrated in an expanded configuration. The expanded configuration houses a valve 3500 that functions in the same manner as a replacement biological valve. The valve 3500 could be expanded within the stent body 3100 and provide a radial force to interact with a collapsing force of a self-expanding central implant structure. Furthermore the stent element 3100 could house a pericardial valve and be expanded at a later date than a first implantation procedure. Or it could be expanded at the time of the first procedure.

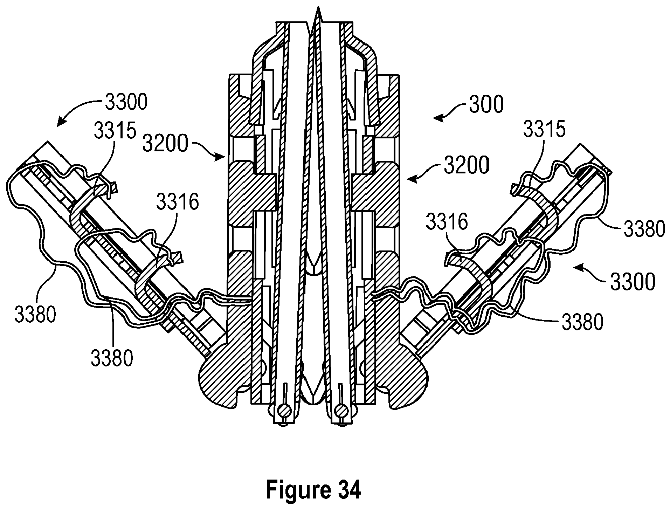

In **Figure 34** a section view of a device 300 is illustrated in the capture ready position. In this embodiment a series of tie lines or cables 3380 connect the gripping elements 3315,3316 to the non-moveable jaw. The orientation of the gripping elements 3315,3316 can be altered by applying or releasing tension in the tensile member formed by the tie lines or cables 3380.

In particular each tie line is attached to the tip of a gripping element 3315,3316 in a manner which does not impede capture of tissue. The tie line then passes through a slot or aperture in the moveable jaw 3300 and passes down the back of the moveable jaw 3300, the tie line then passes to the front of moveable jaw and is terminated at a point in the non-moveable jaw 3200. In the capture ready position of **Figure 34** the tie line is slack. Furthermore the tie line may be concealed within a covering of the moveable jaw 3300. This covering will stop the tie line from deviating to areas of the device that may cause it to become snagged. The gripping elements 3315 and 3316 are resiliently deformable and are configured to be in the exposed position when the tie lines are slack as illustrated in **Figure 34****.**

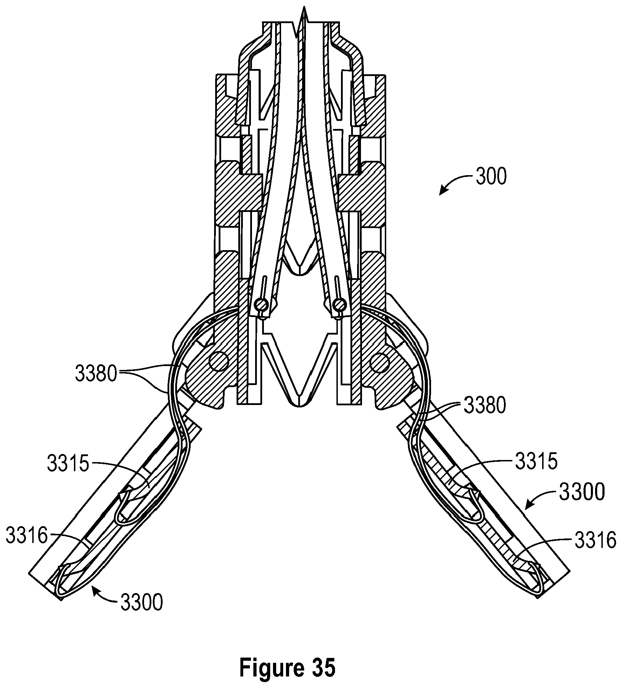

In **Figure 35** a section view of the device is illustrated in the inverted position. In this image the gripping elements 3315, 3316 have been resiliently/elastically deformed by tension in the tie lines 3380. The tension has been imparted into tie line by rotation of the moveable jaw 3300 into an inverted position. Rotation in the opposite direction will relieve the tension in the tie line and the gripping elements will return to their tissue capturing position. In some embodiments only one gripping element may provide sufficient tissue retention characteristics.

The words "comprises/comprising" and the words "having/including" when used herein with reference to the present invention are used to specify the presence of stated features, integers, steps or components but do not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

## Claims

1. A fixation device for fastening to a native heart valve having native valve leaflets, the fixation device comprising:
(a) a device body, and
(b) at least one clamp on the device body, the clamp for engaging and holding a native valve leaflet, the clamp comprising at least one moveable jaw, the moveable jaw having thereon at least one gripping element which is moveable relative to the moveable jaw between a native valve leaflet gripping configuration of the moveable jaw where the gripping element is arranged to grip a native valve leaflet and a passive configuration of the moveable jaw where the gripping element is arranged so that it cannot grip a native valve leaflet.

2. A device according to any preceding claim wherein the device is configured so that movement of the moveable jaw modulates movement of the gripping element relative to the moveable jaw.

3. A device according to any preceding claim wherein the moveable jaw is pivotably mounted on the device body by a pivot and moves by pivotable movement about the pivot.

4. A device according to any preceding claim wherein the device has a clamp closed position and a first clamp open position and the moveable jaw has a first range of movement from the clamp closed position to the first clamp open position through which the jaw is in its native valve gripping configuration; optionally:
wherein the moveable jaw has a second range of movement (distinct from the first range of movement) from the first clamp open position to a second clamp open position, wherein in the second clamp open position the moveable jaw is in its passive configuration where the gripping element is arranged so that it cannot grip a native valve leaflet, for example wherein the device is configured so that during movement through the second range of movement the gripping element is automatically progressively moved from where it is arranged to grip a native valve leaflet to where it is arranged so that it cannot grip a native valve leaflet, further optionally wherein the moveable jaw has a third range of movement (distinct from the first range of movement and the second range of movement) from the second open position to a third open position, through which the moveable jaw is in its passive configuration.

5. A device according to any preceding claim wherein at least a part of the gripping element, for example a gripping surface of the gripping element, stands proud of the (clamping face of the) moveable jaw in the native valve leaflet gripping configuration of the moveable jaw, and/or
wherein, in the passive configuration of the moveable jaw, no part of the gripping element stands proud of the (clamping face of the) moveable jaw.

6. A device according to any preceding claim wherein the position of the gripping element relative to the moveable jaw is changed by raising or lowering the gripping element relative to the jaw, and/or
wherein the position of the gripping element relative to the moveable jaw is changed by pivoting the gripping element relative to the jaw, and/or
wherein the position of the gripping element relative to the moveable jaw is changeable by depression of the gripping element, optionally by depression against the biasing force of a biasing means, and/or
wherein the position of the gripping element relative to the moveable jaw is changeable by deforming the gripping element, optionally wherein the gripping element is resiliently deformable for example by being formed of a resiliently deformable material, e.g. a material such as nitinol.

7. A device according to any preceding claim wherein the moveable jaw has a gripping face and wherein when the gripping element is in the native valve leaflet gripping configuration the gripping element is aligned with the gripping face and wherein when the gripping element is in the passive configuration the gripping element is arranged so that it displaced relative to the gripping face.

8. A device according to any preceding claim wherein the device has a mechanism for changing the position of the gripping element relative to the moveable jaw, desirably a mechanism for automatically changing the position of the gripping element relative to the moveable jaw.

9. A device according to Claim 8 wherein the mechanism comprises a cam surface and movement of the moveable jaw causes the cam surface to impart a force to the gripping element to move it relative to the moveable jaw, and/or wherein the mechanism comprises a cam surface and movement of the moveable jaw causes the cam surface to impart a force to the gripping element to move it relative to the moveable jaw.

10. A device according to any preceding claim further comprising a sliding element which slides on and relative to the moveable jaw and which is slideable in a first direction across the gripping element to move the gripping element relative to the moveable jaw; optionally by depressing the gripping element against a resilient bias, optionally
wherein sliding movement of the sliding element in the first direction causes the gripping element to depress, optionally against the resilient bias, and wherein sliding movement of the sliding element in a second (opposite) direction allows the gripping element to move with the resilient bias, and/or
wherein the gripping element projects through the sliding element, and/or wherein sliding movement of the sliding element in the first direction causes the gripping element to be concealed by the sliding element, optionally wherein sliding movement of the sliding element in a second direction causes the gripping element to be revealed, and/or
wherein the gripping element is resilient biased and the resilient bias is sufficient to resiliently bias the sliding element in a second direction, opposite to the first direction, and/or
wherein the device has a cam surface and movement of the moveable jaw causes the cam surface to impart, in the first direction, a sliding movement to the sliding element, optionally wherein the sliding movement imparted by the cam surface acts against the resilient bias, and/or
wherein the jaw is pivotable about a pivot relative to the device body and optionally wherein the movement of the jaw according to Claim 23 that causes the cam surface to impart, in the first direction, a sliding movement to the sliding element, is a pivoting movement about the pivot.

11. A device according to any preceding claim wherein the clamp is formed by the moveable jaw and a non-moving part of the device body, for example a non-moving jaw, optionally wherein the gripping element projects into the non-moveable part.

12. A device according to any of Claims 4 to 11 wherein the moveable jaw moves by pivoting about a pivot from the closed position to the first, second and third open positions and the second open position is at least 110 degrees of rotation away from the closed position, and/or wherein the second open position is at least 120 degrees of rotation away from the closed position.

13. A device according to any preceding claim wherein the gripping element is curved, and/or
wherein the gripping element is a piercing element, optionally wherein, in the native valve leaflet gripping configuration of the moveable jaw the piercing element is arranged to pierce a native valve leaflet, and in the passive configuration of the moveable jaw the piercing element is arranged so that it cannot pierce a native valve leaflet.

14. A device according to any preceding claim wherein the device comprises a tie line or cable and movement of the moveable jaw causes the cable to impart a force, for example impart a tension force in the cable, to the gripping element to move it relative to the moveable jaw; optionally wherein pivoting movement of the moveable jaw causes the cable to impart a force, for example impart a tension force in the cable, to the gripping element to move it relative to the moveable jaw.

15. A fixation device for fastening to a native heart valve having native valve leaflets, the fixation device comprising:
(a) a device body,
(b) at least one clamp on the device body, the clamp for engaging and holding a native valve leaflet, the clamp comprising at least one jaw, the jaw having thereon at least one gripping element, the gripping element being resiliently deformable,
(c) a contact on the device body; and
the gripping element and the contact being arranged so that when the clamp grips a native valve leaflet, deformation of the gripping element occurs causing the gripping element to move against the contact.

16. A fixation device according to Claim 15 wherein the jaw is a moveable jaw,
and/or
wherein the gripping element is moveable relative to the jaw between a native valve leaflet gripping configuration of the jaw where the gripping element is arranged to grip a native valve leaflet and a passive configuration of the jaw where the gripping element is arranged so that it cannot grip a native valve leaflet.

17. An implant device for implantation into the animal body, the implant comprising
(a) a device body;
(b) at least one moveable securing element on the device body which is moveable relative to the device body for securing the device in position by gripping tissue;
(c) at least one sensor on the device body or the securing element for indicating that the securing element has gripped tissue;
wherein the at least one sensor is detachable so that it is removable after the device has been implanted.

18. An implant device according to Claim 17 wherein the at least one sensor has an electrical wire electrically connected thereto and the sensor is detachable by applying a force such as a tensile force to the electrical wire, and/or wherein the at least one sensor is positioned in a recess in the device body or the securing element and is removable from the recess, and/or
wherein the sensor is releasably secured to the device body or the securing element, such as by bonding, for example bonding using a debondable cured adhesive or bonding using an adhesive that forms a bond which fails.
